# FASCICULE DE BREVET EUROPEEN

(11) **EP 3 538 258 B1**
(45) Date de publication et mention de la délivrance du brevet: **04.08.2021**
(21) Numéro de dépôt: 17804245.3
(22) Date de dépôt: 13.11.2017
(51) Int. Cl.: B01J 13/00

(54) **SUSPENSION COLLOÏDALE DE NANOPARTICULES DE FLUORURE D'ELEMENT ALCALINO-TERREUX FONCTIONNALISEES**
KOLLOIDALE SUSPENSION VON FUNKTIONALISIERTEN NANOPARTIKELN EINES FLUORIDS EINES ERDALKALIELEMENTS
COLLOIDAL SUSPENSION OF FUNCTIONALISED NANOPARTICLES OF A FLUORIDE OF AN ALKALINE-EARTH ELEMENT

(30) Priorité: 14.11.2016 FR 1660945
(43) Date de publication de la demande: 18.09.2019
(73) Titulaire: Mathym, 69410 Champagne-Au-Mont-D'or (FR)
(72) Inventeur: FAURE, Anne-Charlotte Françoise, 69100 Vileurbanne (FR); ALBERICI, Julien, 69004 Lyon (FR)
(74) Mandataire: Schmidt, Martin Peter
(86) Numéro de dépôt international: PCT/FR2017/053087
(87) Numéro de publication internationale: WO 2018/087495

(56) Documents cités:
- EP-A1- 2 253 590
- EP-A1- 2 676 938
- FR-A1- 2 946 973
- FUJIHARA S ET AL: "Formation of LaF3 microcrystals in sol-gel silica", JOURNAL OF NON-CRYSTALLINE SOLIDS, NORTH-HOLLAND PHYSICS PUBLISHING. AMSTERDAM, NL, vol. 244, no. 2-3, 2 mars 1999 (1999-03-02), pages 267-274, XP004166727, ISSN: 0022-3093, DOI: 10.1016/S0022-3093(99)00009-5
- FUJIHARA S ET AL: "Influence of solution composition on the formation of SiO2/LaF3 composites in the sol-gel process", JOURNAL OF MATERIALS SCIENCE, KLUWER ACADEMIC PUBLISHERS, DORDRECHT, vol. 35, no. 11, 1 juin 2000 (2000-06-01), pages 2763-2767, XP002573381, ISSN: 0022-2461, DOI: 10.1023/A:1004726630820 [extrait le 2004-11-01]

## Description

### Domaine technique de l'invention

L'invention concerne le domaine de la chimie minérale, à savoir le domaine des fluorures des éléments alcalino-terreux. Elle concerne plus particulièrement des suspensions colloïdales de fluorure d'éléments alcalino-terreux, et notamment un procédé de synthèse de nanoparticules de fluorure d'éléments alcalino-terreux fonctionnalisées et leur utilisation, sous forme de suspension colloïdale, pour la préparation de produits de traitement prophylactique ou curatif des dents.

### Etat de la technique

On connait les verres de fluorure de magnésium ou de calcium préparés à partir d'un gel vitreux de fluorure de magnésium ou de calcium ; ce gel peut être obtenu à partir de méthoxyde de magnésium et d'une solution anhydre de fluorure d'hydrogène dans de l'éther, comme cela est décrit dans EP 1 585 542 A1 (Université de Berlin) et EP 1 732 853 B1 (E. Kemnitz et al.). Le mécanisme réactionnel est discuté dans la publication « Non-aqueous sol-gel synthesis of nano-structured metal fluorides » par S. Rüdiger et al., parue dans Journal of Fluorine Chemistry 128 (2007), p. 353-368.

Les suspensions de nanoparticules de fluorures de magnésium ou calcium ont été proposées comme point de départ pour le dépôt de couches optiques. Pour cette application, on cherche à former une couche aussi transparente que possible dans le domaine spectral visé. Pour obtenir une haute transparence optique, la couche ne doit ni absorber ni diffuser la lumière. La diffusion de la lumière est minimisée si les particules ou leurs agglomérats sont de petite taille (i.e. d'un diamètre ne dépassant pas la longueur d'onde de la lumière). L'absorption de la lumière est provoquée notamment par la présence de produits carbonés ; par conséquent, les particules ne doivent pas comporter des contaminations organiques à leur surface. Par ailleurs, les propriétés optiques de la couche doivent être stables sur la durée d'utilisation de l'élément optique sur lequel elle a été appliquée; à ce titre, il n'est pas souhaitable que la structure de la couche évolue, notamment par cristallisation.

On connaît plusieurs procédés de fabrication de nanoparticules de fluorure d'élément alcalino-terreux.

EP 2 253 590 A1 (Central Glass Company) décrit un procédé pour produire des nanoparticules de fluorure de magnésium hydroxyde nanocristallin pour couches optiques.

FR 2 946 973 A1 divulgue un procédé de préparation de nanoparticules à base de fluorure de terre rare.

EP 2 676 938 A1 (Nanofluor GmbH) décrit un procédé de fabrication de nanoparticules de CaF₂ dans lequel on fait réagir un équivalent d'un précurseur de calcium dans un solvant non-aqueux avec deux équivalents de HF anhydre. Ce procédé conduit à un sol comprenant des particules de CaF₂ d'une taille inférieure à 50 nm. Ces nanoparticules sont amorphes. Ce procédé présente deux inconvénients majeurs qui posent un problème pratique lorsqu'il s'agira de le transposer à une échelle industrielle.

Le premier inconvénient est lié à l'emploi de fluorure d'hydrogène (HF) anhydre. Le HF anhydre résulte en règle générale de la saturation d'un solvant non aqueux, tel que le méthanol, par du HF gazeux à l'aide d'un bulleur : il nécessite l'utilisation de HF gazeux approvisionné en bouteilles. Or, ce gaz est toxique, très réactif et, en particulier, extrêmement corrosif en présence de traces d'humidité. Il est incompatible avec de nombreux matériaux utilisés en génie chimique et nécessite en particulier une robinetterie spécifique qui ne comporte pas de pièce en cuivre, laiton ou aluminium en contact avec le gaz. Tout changement de bouteille de gaz HF représente une situation à risque. Et finalement, les effluents de HF gazeux nécessitent un traitement particulier.

Le deuxième inconvénient est que la réaction décrite dans EP 2 676 938 est très lente : les exemples donnés dans ce document indiquent une durée de réaction comprise entre trois et sept jours sous agitation.

Il existe cependant un besoin de disposer d'un procédé de fabrication de suspensions de nanoparticules qui soit plus rapide, moins dangereux et qui ne génère pas d'effluents (notamment gazeux) réactifs et difficiles à traiter.

Il existe également un besoin de disposer de nanoparticules de fluorure d'élément alcalino-terreux fonctionnalisées, c'est-à-dire dont les propriétés de surface ont été modifiées par la présence de groupements organiques appropriés. D'une manière générale, les fluorures d'élément alcalino-terreux sont peu solubles, voire insolubles dans l'eau ; pour toute utilisation médicale, il faut veiller à ne pas les contaminer avec des traces de solvant indésirable dans le produit fini.

Il existe notamment un besoin de disposer de suspensions colloïdales aqueuses ou éthanoliques de telles nanoparticules fonctionnalisées. Ces suspensions colloïdales doivent être stables, même à fort extrait sec (par exemple supérieur à 5% massiques, et de préférence supérieure à 10% massiques). Idéalement, elles comprennent des nanoparticules de petite taille, et notamment une taille donnée en D50 inférieure à 300 nm, de préférence inférieure à 200 nm, et encore plus préférentiellement inférieure à 100 nm. Leur composition chimique, leur cristallinité et leur pureté doivent être maitrisées.

Au vue de ce qui précède, le problème principal que la présente invention cherche à résoudre est de proposer un procédé simple et rapide permettant l'obtention directe de suspensions colloïdales de nanoparticules de fluorure d'élément alcalino-terreux individualisées (i.e. non agglomérées), fonctionnalisées et stables, un procédé qui soit aussi peu dangereux que possible et qui ne génère que des effluents faciles à traiter.

La présente invention a aussi pour objet de fournir un procédé de fabrication de suspensions colloïdales de nanoparticules de fluorure d'alcalino-terreux qui soit simple, sûr, rapide, facile à mettre en oeuvre, peu coûteux et pouvant s'affranchir de l'emploi de solvant toxique.

Un autre objectif de l'invention est de proposer des suspensions colloïdales de nanoparticules de fluorure d'alcalino-terreux prêtes à l'emploi et facilement fonctionnalisables.

Un autre objectif de l'invention est de proposer des suspensions colloïdales de nanoparticules de fluorure de calcium fonctionnalisées, pouvant notamment être utilisées dans la fabrication de produits de traitement prophylactique ou curatif des dents.

### Objets de l'invention

La présente invention concerne une suspension colloïdale de nanoparticules de fluorure d'élément alcalino-terreux dans laquelle lesdites nanoparticules ont une taille donnée en D50 généralement inférieure à 300 nm, de préférence inférieure à 200 nm, et encore plus préférentiellement inférieure à 100 nm. On préfère que la taille D50 soit comprise entre 5 nm et 30 nm, et de préférence entre 5 nm et 20 nm.

Ledit élément alcalino-terreux peut notamment être le magnésium, le calcium, le baryum, le strontium, le béryllium, le radium ; le magnésium, le calcium et le strontium sont préférés.

Selon une caractéristique essentielle de l'invention, lesdites particules sont fonctionnalisées par au moins un type de molécules organiques.

Dans un mode de réalisation, le solvant dispersant les nanoparticules de fluorure d'élément alcalino-terreux fonctionnalisées ou non fonctionnalisées est choisi parmi les hydrocarbures, les hydrocarbures halogénés, l'eau, les alcools, les éthers, les glycols, les éthers de glycol, les aldéhydes, les cétones, les acides carboxyliques, les esters, les esters de carbonate, les amines, les amides, les thiols, les solvants organosulfurés, les monomères acryliques et méthacryliques, les monomères vinyliques, les monomères époxydes, les huiles silicone, les organosilanes, les polycarbosilanes et les alkoxysilanes.

Un objet de l'invention est un procédé de préparation d'une suspension colloïdale de nanoparticules de fluorure d'élément alcalino-terreux dans lequel :
(a) on prépare une solution, de préférence aqueuse, d'un sel de l'élément alcalino-terreux correspondant,
(b) on prépare une solution d'un complexe de transfert de charge de formule (I) 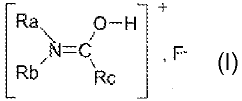 par réaction d'un amide de formule II 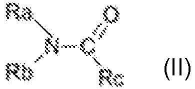 avec une source d'ions fluorure, de préférence une solution d'acide fluorhydrique, du fluorure de tétrabutylammonium, du trihydrofluorure de triéthylamine ou de l'acide fluorosilicique,
   où : Ra et Rb, identiques ou différents, représentent, chacun indépendamment l'un de l'autre, un groupe (C1-C6)alkyle, (C3-C7)cycloalkyle, phényle ou hétérocycloalkyle de 5 ou 6 chainons, ledit groupe pouvant éventuellement être substitué, ou bien Ra et Rb sont liés entre eux et forment une chaine alkylène, comprenant de 2 à 6 atomes de carbone, éventuellement substituée, et Rc représente un atome d'hydrogène ou un groupe (C1-C6)alkyle, (C3-C7)cycloalkyle, phényle ou hétérocycloalkyle de 5 ou 6 chainons, ledit groupe pouvant éventuellement être substitué, ou bien Rb et Rc sont liés entre eux et forment une chaine alkyle, hétérocycloalkye ou alkylène, comprenant de 2 à 5 atomes de carbone, éventuellement substituée,
(c) on met en contact, dans un solvant ou mélange de solvants, la solution d'un sel de l'élément alcalino-terreux correspondant obtenue à l'étape a) et la solution d'un complexe de transfert de charge de formule (I) obtenue à l'étape b) 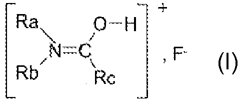
(d) on laisse réagir, éventuellement en chauffant dans un récipient hermétiquement fermé ou non, le mélange obtenu à l'étape (c) de manière à obtenir une suspension colloïdale de nanoparticules de fluorure d'élément alcalino-terreux,
(e) optionnellement, on lave la suspension colloïdale de nanoparticules de fluorure d'élément alcalino-terreux, de préférence par au moins un cycle de centrifugation/ redispersion dans un solvant approprié jusqu'à ce que le surnageant soit limpide et incolore, étant entendu que le dernier solvant de redispersion est choisi en fonction du solvant d'utilisation de la suspension colloïdale de nanoparticules de fluorure d'élément alcalino-terreux, sachant que dans ce procédé les étapes (a) et (b) peuvent être inversées.

Selon un mode de réalisation de l'invention, c'est l'amide de formule II ou son produit de dégradation qui assure la fonctionnalisation des nanoparticules de fluorure d'élément alcalino-terreux. De manière préférée, ledit amide de formule II est une pyrrolidone, de préférence substituée et encore plus préférentiellement N-substituée. On peut utiliser par exemple la 2-pyrrolidone et la N-méthyl-2-pyrrolidone. D'autres modes de fonctionnalisation peuvent être utilisés, comme cela sera expliqué ci-dessous.

Un autre objet de l'invention concerne un procédé de préparation d'une suspension colloïdale de nanoparticules de fluorure d'élément alcalino-terreux fonctionnalisées dans lequel :
(i) on prépare une suspension colloïdale de nanoparticules de fluorure d'élément alcalino-terreux comme indiqué précédemment,
(ii) on greffe à la surface des nanoparticules de fluorure d'élément alcalino-terreux une molécule présentant une structure du type A-Sp-Z dans laquelle :
   i. A est une fonction chimique assurant l'accrochage de la molécule à la surface,
   ii. Sp est un groupe espaceur,
   iii. Z est une fonction chimique permettant de modifier le caractère de surface des nanoparticules
(iii) optionnellement, on lave la suspension colloïdale de nanoparticules de fluorure d'élément alcalino-terreux fonctionnalisées, de préférence par au moins un cycle de centrifugation/redispersion dans un solvant approprié jusqu'à ce que le surnageant soit exempt de molécules fonctionnalisantes libres, étant entendu que le dernier solvant de redispersion est choisi en fonction du solvant d'utilisation de la suspension colloïdale de nanoparticules de fluorure d'élément alcalino-terreux fonctionnalisées. Dans un mode de réalisation, le dernier solvant de redispersion est identique au solvant d'utilisation de la suspension, ou au moins miscible avec ce dernier.

Dans un mode de réalisation particulier, on reconcentre la suspension colloïdale de nanoparticules de fluorure d'élément alcalino-terreux fonctionnalisées jusqu'à atteindre l'extrait sec désiré, de préférence jusqu'à atteindre un extrait sec d'au moins 5 %, d'au moins 10%, d'au moins 15%, d'au moins 18%, voire d'au moins 20%, d'au moins 25%, d'au moins 30%, d'au moins 35 %, d'au moins 40 %, d'au moins 45 % ou même d'au moins 50 % massiques.

Avantageusement, l'élément alcalino-terreux est choisi parmi le magnésium, le calcium et le strontium.

Si la suspension colloïdale de nanoparticules de fluorure d'élément alcalino-terreux est destinée à être utilisée pour les applications médicales, et en particulier intracorporelles comme en médecine dentaire, on préfère que le(s) solvant(s) utilisé(s) pour la réaction entre le sel de l'élément alcalino-terreux et le complexe de transfert de charge soi(en)t non toxique(s), et que la phase liquide dispersant les nanoparticules soit choisie parmi des solvants non toxiques compatibles avec leur application. Par « solvant non toxique », on entend tout solvant présentant peu ou pas de risques pour la santé, tel que l'eau, l'éthanol, et les mélanges eau - éthanol.

L'invention a également pour objet une suspension colloïdale de nanoparticules de fluorure d'élément alcalino-terreux susceptible d'être obtenue par les procédés précédemment décrits.

Avantageusement, la suspension colloïdale de nanoparticules de fluorure d'élément alcalino-terreux est utilisée dans la fabrication d'un matériau de reminéralisation dentaire.

L'invention a également pour objet l'utilisation d'une suspension colloïdale de nanoparticules de fluorure d'élément alcalino-terreux dans la fabrication d'un matériau de traitement prophylactique ou curatif des dents, et notamment pour la reminéralisation dentaire.

L'invention a également pour objet une composition comprenant au moins une suspension colloïdale de nanoparticules de fluorure d'élément alcalino-terreux fonctionnalisées.

### Description des figures

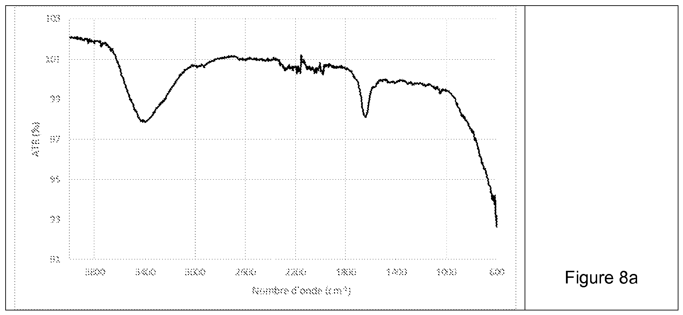

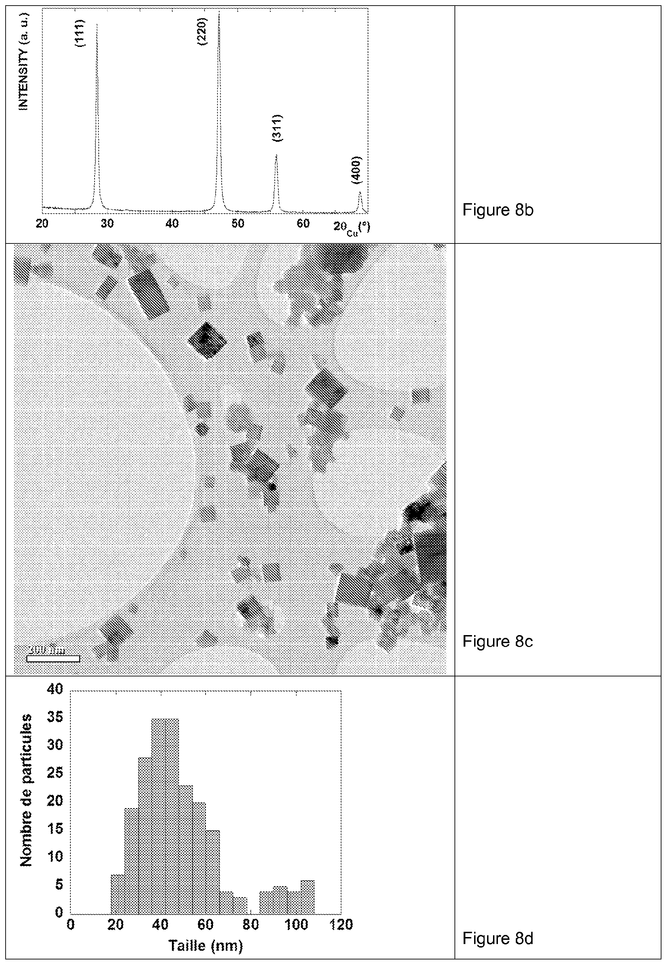

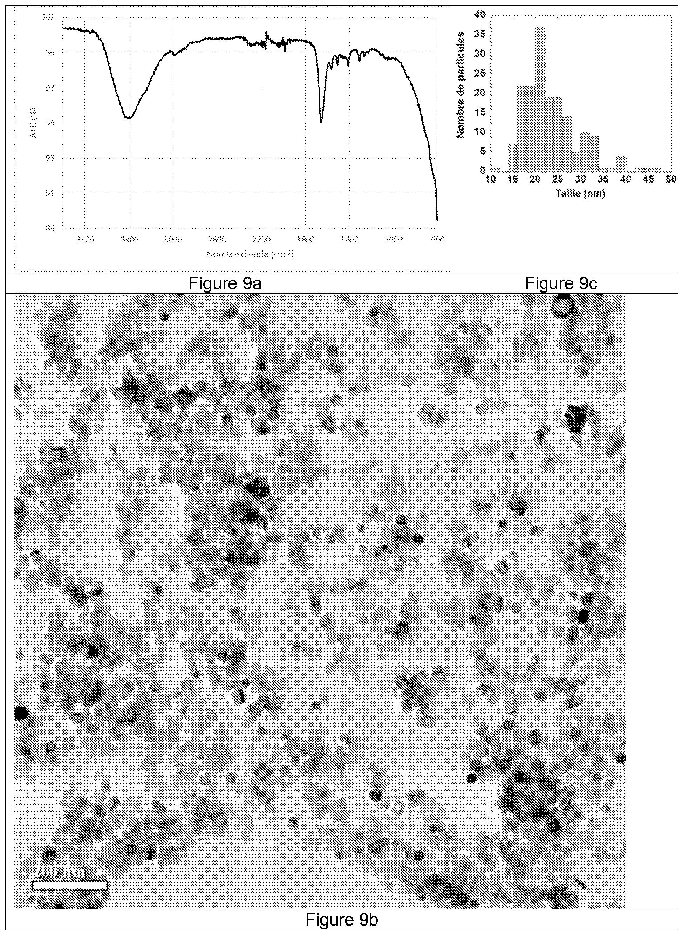

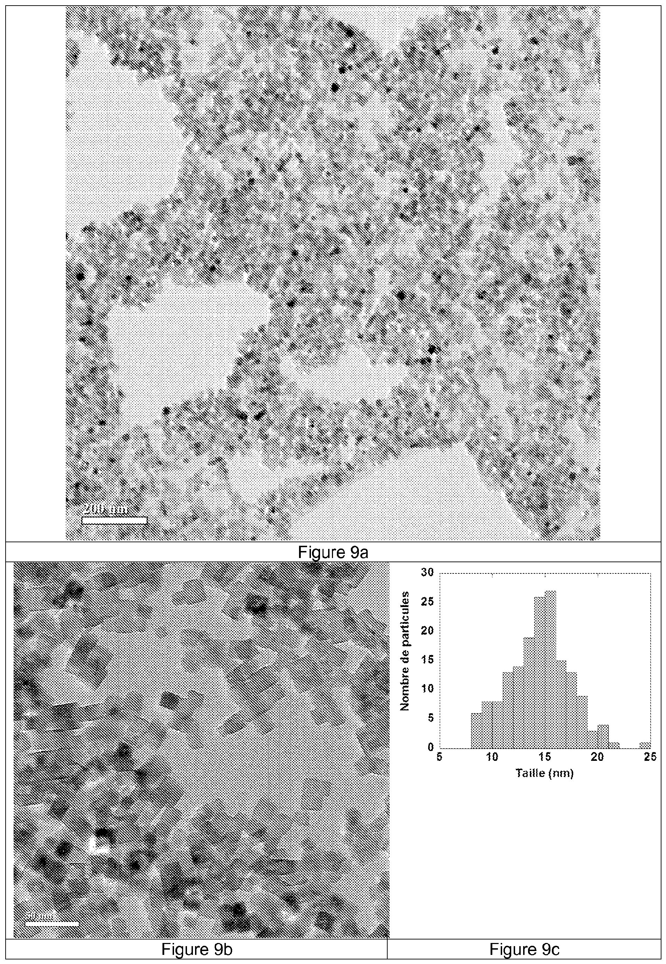

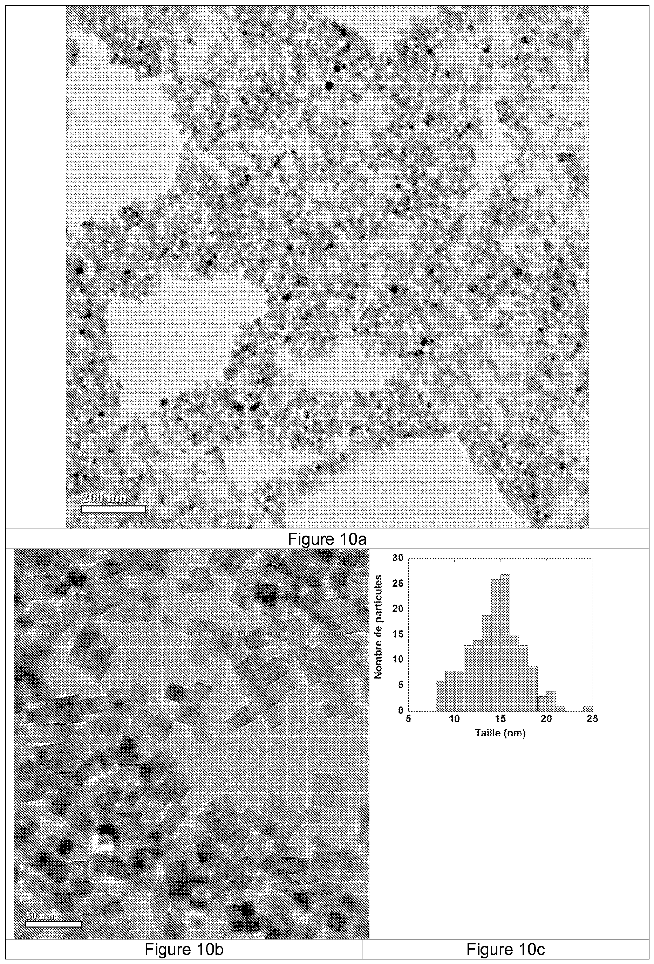

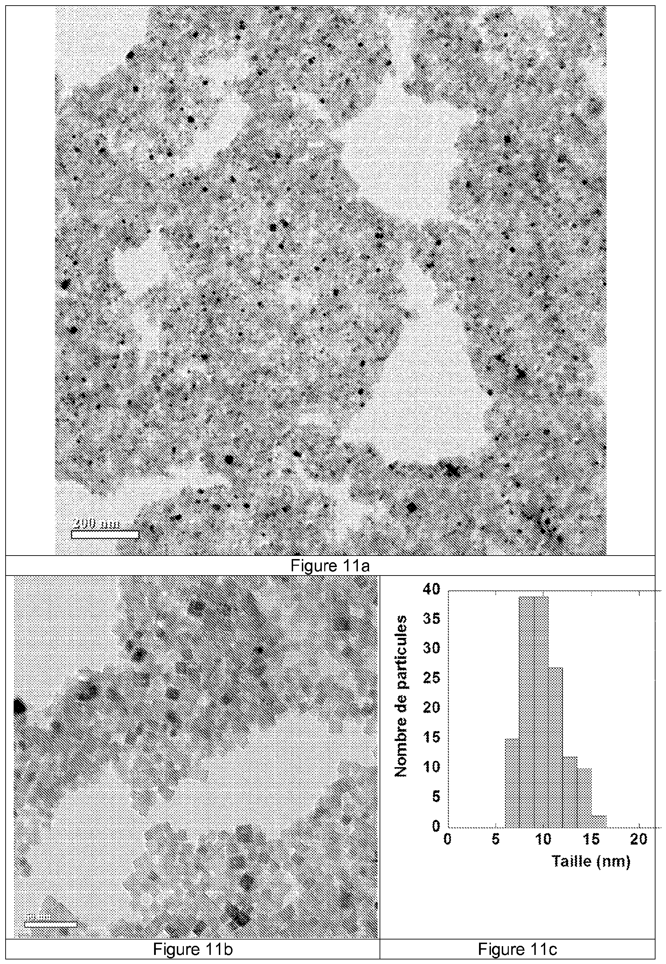

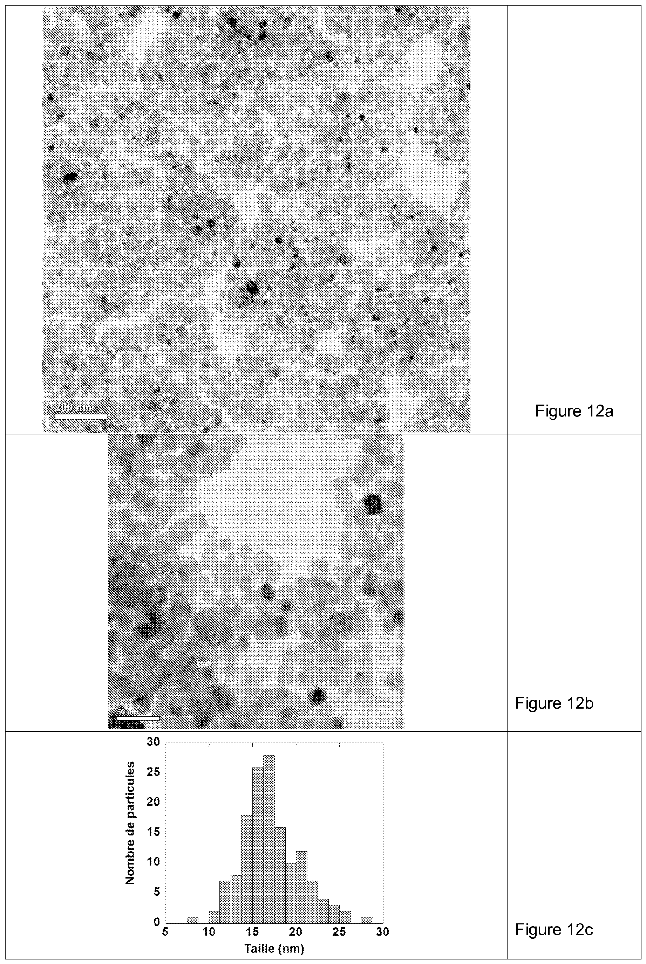

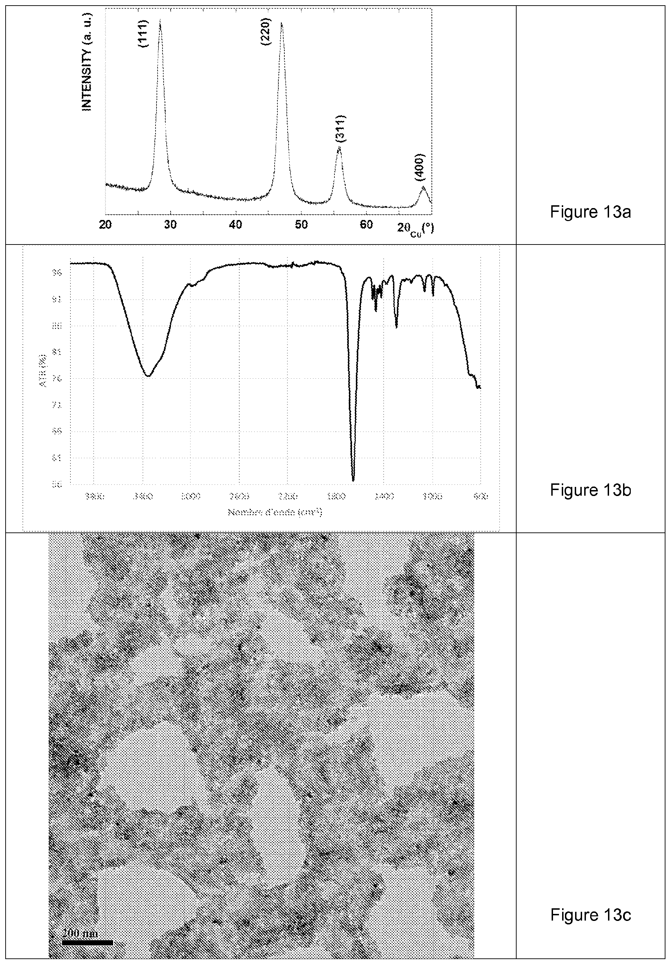

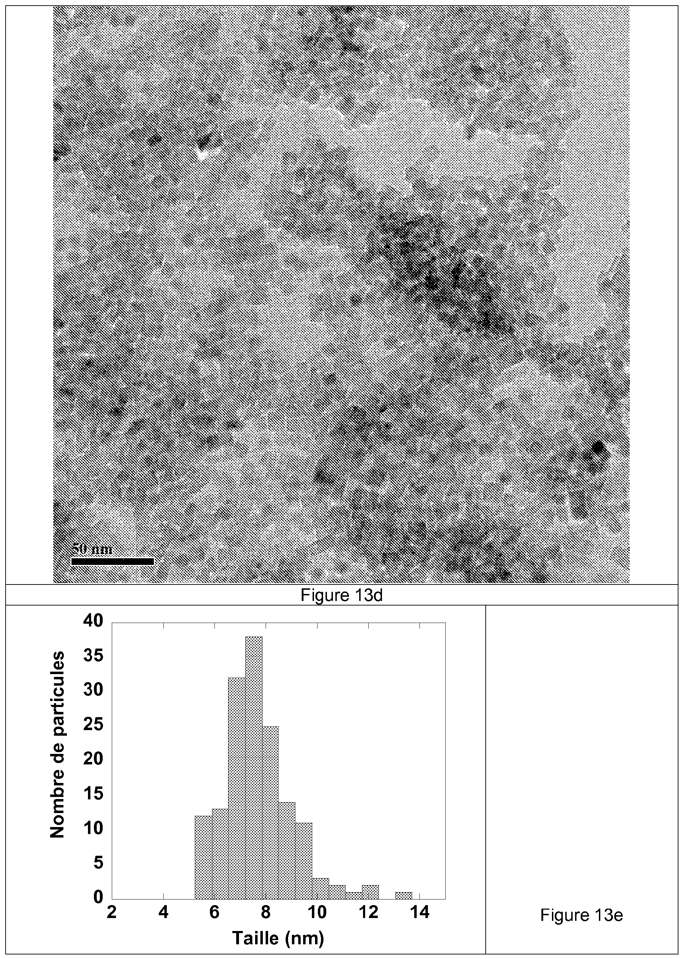

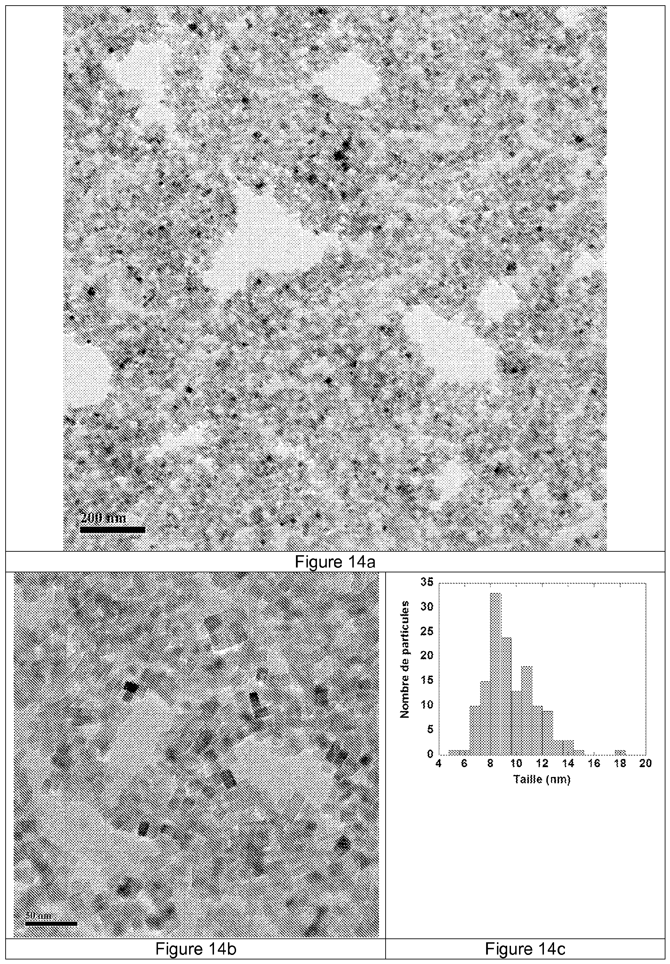

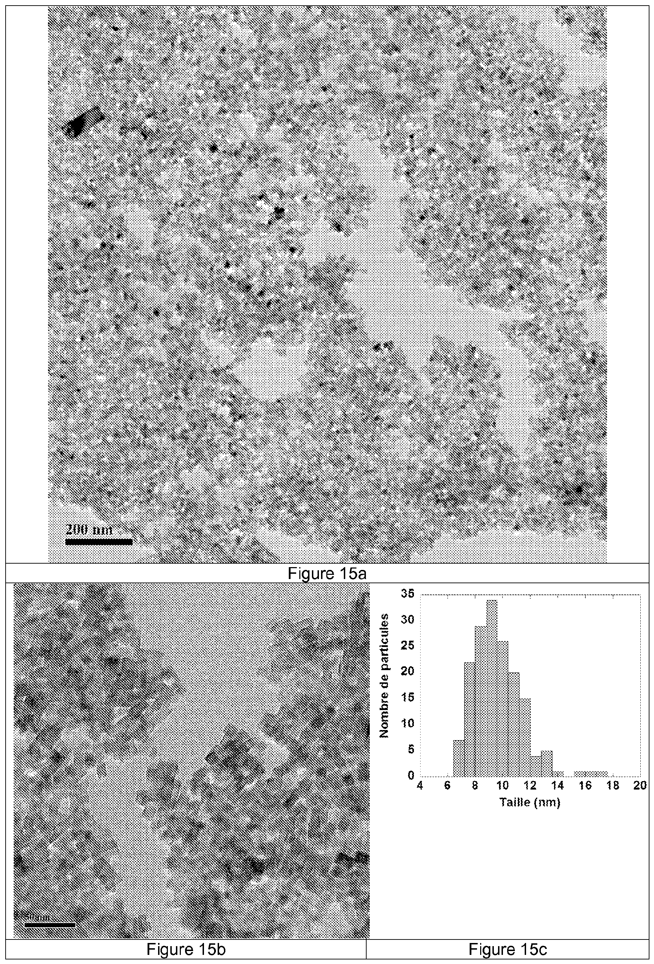

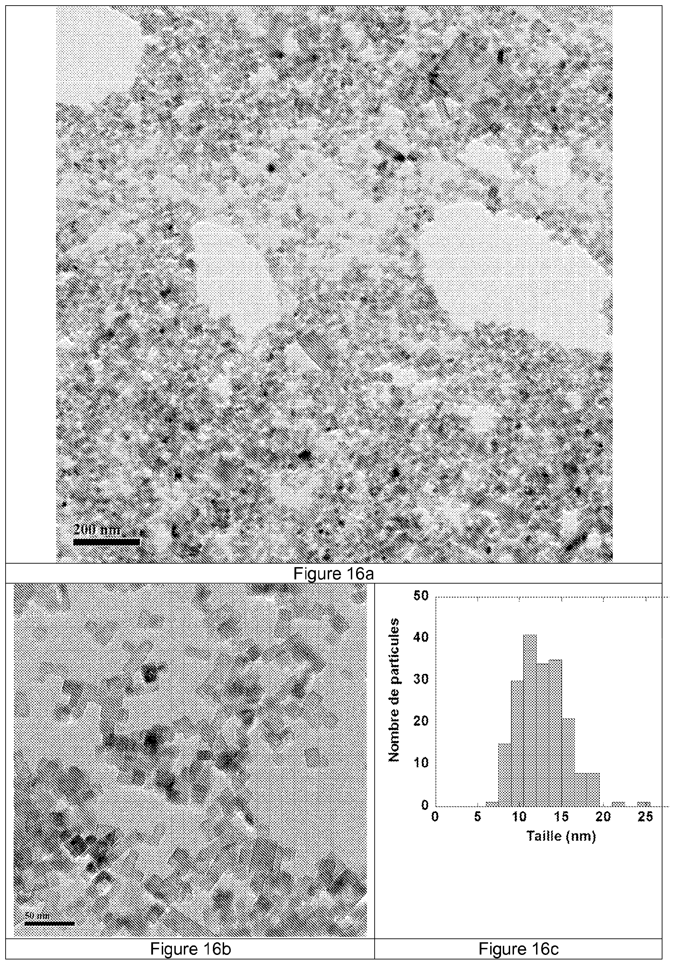

Les figures 1 à 17 illustrent différents aspects de modes de réalisation de l'invention, sans pour autant limiter sa portée. Les figures 1 et 2 se réfèrent à l'exemple 1-1. La figure 3 se réfère à l'exemple 1-2, la figure 4 à l'exemple 3, la figure 5 à l'exemple 4, les figures 6a et 6b à l'exemple 5. Les figures 7 à 17 se réfèrent respectivement aux exemples 6, 7, 9, 11, 14, 15, 16, 17, 19, 20 et 25.
La figure 1a montre une image de microscopie électronique à transmission (MET) de nanoparticules de CaF₂ fonctionnalisées obtenues par le procédé selon l'invention, en utilisant le solvant NMP. La figure 1b montre la distribution en taille des nanoparticules obtenues par le procédé; on note une valeur moyenne de 21,1 nm avec un écart-type de 8,2 nm. La figure 1c montre le diagramme de diffraction des Rayons X (Cu K_{α} du cuivre) de la poudre.
La figure 2 montre pour ces mêmes nanoparticules le résultat d'une mesure de leur taille par diffusion dynamique de la lumière (DLS - Dynamic Light Scattering) réalisée, pour la figure 2a, sur une suspension aqueuse et, pour la figure 2b, sur une suspension éthanolique.
Les figures 4 et 5 montrent chacune une image MET des nanoparticules de CaF₂ obtenues à l'exemple référencé ci-dessus.
Les figures 5 et 6a montrent chacune une image MET des nanoparticules de MgF₂ obtenues dans l'exemple référencé ci-dessus ; la figure 6b montre le diagramme de diffraction des Rayons X (Cu K_{α} du cuivre) de la poudre observée sur la figure 6a.
Les figures 7 à 17 montrent, comme cela sera identifié facilement par l'homme du métier, des diagrammes de diffraction des Rayons X, des spectres d'absorption infrarouge, des images MET, des histogrammes de la mesure de la taille des particules obtenue par analyse d'images à partir des micrographies MET.

### Description détaillée

Dans le cadre du présent document, la taille d'une particule est définie par sa plus grande dimension. Par « nanoparticule », on entend toute particule ou objet de taille nanométrique présentant au moins une de ses dimensions inférieure ou égale à 100 nm. Cependant, l'invention est applicable aussi à des particules de taille supérieure à 100 nm, mais de préférence inférieure à 300 nm.

La « taille de particules » ou « taille moyenne de particules » d'une poudre ou d'un ensemble des particules est donnée en D50 et peut notamment être mesurée par diffusion dynamique de la lumière ou par analyse d'images obtenues par microscopie électronique ; ces deux méthodes ne donnent cependant pas le même résultat (comme cela sera expliqué ci-dessous), et il est nécessaire de se référer à l'une ou l'autre de ces deux méthodes lorsque l'on indique des valeurs numériques.

La présente invention concerne des suspensions colloïdales de fluorure d'élément alcalino-terreux sous forme de nanoparticules, un procédé de synthèse de nanoparticules de fluorure d'élément alcalino-terreux, la fonctionnalisation de ces nanoparticules et leurs utilisations comme nanocharges dans des compositions, notamment dans des compositions dentaires, de préférence dans des matériaux de reminéralisation dentaire. Les particules selon l'invention sont stables au stockage, conçues pour un stockage et une utilisation à des extraits secs supérieurs à 5% massique.

### 1) Synthèse de nanoparticules de fluorure d'élément alcalino-terreux fonctionnalisées

L'invention concerne un procédé de préparation de nanoparticules composées, au moins en partie, d'un fluorure d'élément alcalino-terreux. Ce procédé est caractérisé en ce que ledit fluorure est obtenu, en solution, à partir d'un sel d'élément alcalino-terreux correspondant et d'un complexe de transfert de charge obtenu par la réaction d'un amide de formule II avec une source d'ions fluorure telle que de l'acide fluorhydrique: 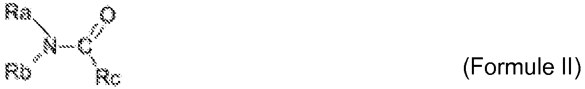 où :
- Ra et Rb, identiques ou différents, représentent, chacun indépendamment l'un de l'autre, un groupe (C₁-C₆)alkyle, (C₃-C₇)cycloalkyle, phényle ou hétérocycloalkyle de 5 ou 6 chaînons, ledit groupe pouvant éventuellement être substitué, ou bien Ra et Rb sont liés entre eux et forment une chaine alkylène, comprenant de 2 à 6 atomes de carbone, éventuellement substituée, et
- Rc représente un atome d'hydrogène ou un groupe (C₁-C₆)alkyle, (C₃-C₇)cycloalkyle, phényle ou hétérocycloalkyle de 5 ou 6 chaînons, ledit groupe pouvant éventuellement être substitué, ou bien Rb et Rc sont liés entre eux et forment une chaine alkylène, comprenant de 2 à 5 atomes de carbone, éventuellement substituée.

Par (C₁-C₆)alkyle, on entend un groupe hydrocarboné saturé, linéaire ou ramifié, comprenant de 1 à 6 atomes de carbone, tels que les groupes méthyle, éthyle, iso-propyle, n-propyle, sec-butyle, tett-butyle, n-butyle, n-pentyle, n- hexyle.

Par chaine alkylène comprenant de 2 à 6 atomes de carbone, on entend une chaine -(CH₂)ₚ- avec p = 2, 3, 4, 5 ou 6.

Par (C₃-C₇)cycloalkyle, on entend un groupe hydrocarboné saturé cyclique, comprenant de 3 à 7 atomes de carbone.

Par hétérocycloalkyle, on entend un groupe cycloalkyle tel que défini ci-dessus dans lequel l'un au moins des atomes de carbone a été remplacé par un hétéroatome, du type N ou S, et notamment un groupe pyrrolidine ou pipéridine.

Par source d'ions fluorure F⁻, on entend tout composé chimique dont la dissociation fournit des ions fluorure, tel que l'acide fluorhydrique (HF), le fluorure de tétrabutylammonium, le trihydrofluorure de triéthylamine ou l'acide fluorosilicique. Avantageusement, la source d'ions fluorure employée est l'acide fluorhydrique.

Dans le cas où un des groupes (C₁-C₆)alkyle, (C₃-C₇)cycloalkyle, ou phényle, sera substitué, il pourra être substitué par un ou plusieurs substituants, notamment choisis parmi les halogènes (F, Cl, Br, I), les groupes (C₁-C₆) alkyle, phényle, hydroxy (-OH), (C₁-C₆) alkylhydroxy (-(C₁-C₆)alkylOH). Néanmoins, de préférence, ces groupes seront non substitués, leur préparation étant plus aisée dans ce cas.

Bien entendu, dans le complexe de transfert de charge de formule (I), lorsque Ra et Rb seront liés entre eux pour former une chaine alkylène, Rc représentera un atome d'hydrogène ou un groupe (C₁-C₆)alkyle, (C₃-C₇)cycloalkyle, phényle ou hétérocycloalkyle de 5 ou 6 chaînons, éventuellement substitué et Rc ne pourra pas être lié à Rb.

De même, lorsque Rb et Rc seront liés entre eux pour former une chaîne alkylène, Ra représentera, un groupe (C₁-C₆)alkyle, (C₃-C₇)cycloalkyle, phényle ou hétérocycloalkyle à 5 ou 6 chaînons, éventuellement substitué, et Ra ne pourra pas être lié à Rb.

A titre d'exemple, le composé de formule II peut être une pyrrolidone, et en particulier un N-alkyl-pyrrolidone, dans laquelle le reste alkyl lié à l'azote est avantageusement méthyl, éthyl ou propyl, et de préférence un méthyl.

Dans le cadre du procédé selon l'invention, le complexe de transfert de charge est préparé séparément avant toute réaction avec un sel d'élément alcalino-terreux.

Selon l'invention, la réaction entre le sel de l'élément alcalino-terreux et le complexe de transfert de charge est réalisée dans un mélange d'un premier solvant (appelé ici « solvant 1 »), d'un amide, et éventuellement d'un deuxième solvant (appelé ici « solvant 2 »), le solvant 2 étant différent de l'amide. Le solvant 1 doit être choisi pour permettre de dissoudre le sel d'élément alcalino-terreux utilisé. Le sel d'élément alcalino-terreux utilisé est, par exemple, choisi parmi les chlorures, nitrates et alkoxydes, notamment en tenant compte de sa solubilité dans le milieu réactionnel choisi ; les chlorures sont préférés, sachant que les nitrates présentent un risque d'explosion, et sachant que les alkoxydes sont chers et difficilement manipulables à cause de leur réactivité en présence d'humidité.

Le solvant 2 est utilisé pour dissoudre l'amide si ce dernier n'est pas liquide.

Le solvant du mélange utilisé pour la réaction entre le sel d'élément alcalino-terreux et le complexe de transfert de charge peut correspondre au solvant utilisé pour préparer le complexe de transfert de charge.

Par exemple, le complexe de transfert de charge peut être obtenu par réaction de HF, comme source d'ions fluorure, et de diméthylformamide, conduisant à la formation d'un complexe de transfert de charge dans lequel Ra=Rb=méthyle et Rc=H. Le complexe de transfert de charge peut également être obtenu par réaction de HF et de diméthylacétamide, conduisant à la formation d'un complexe de transfert de charge dans lequel Ra=Rb=Rc=méthyle. Selon une alternative, le complexe de transfert de charge peut être obtenu par réaction de HF et de N-méthylpyrrolidinone, conduisant à la formation d'un complexe de transfert de charge dans lequel Ra= méthyle et -Rc-Rb-=-(CH₂)₃-. La préparation du complexe de transfert de charge pourra être effectuée, notamment à température ambiante et sous pression atmosphérique, et ce en l'absence de solvant additionnel, le diméthylformamide, le diméthylacétamide ou la N-méthylpyrrolidinone jouant le rôle à la fois de réactif et de solvant. De préférence, par la suite, le diméthylformamide, le diméthylacétamide ou la N-méthylpyrrolidinone joue également le rôle de solvant pour la réaction entre le sel d'élément alcalino-terreux et le complexe de transfert de charge. Une partie du solvant sert à stabiliser la suspension colloïdale qui va être obtenue, par effet de complexation à la surface des nanoparticules. De façon avantageuse, la réaction entre le sel d'alcalino-terreux et le complexe de transfert de charge est réalisée dans un mélange de solvants comprenant le solvant 1, l'amide si ce dernier joue le rôle de solvant et éventuellement le solvant 2. Avantageusement, le mélange de solvant contient un solvant protique, tel que l'eau, le méthanol, l'éthanol ou l'isopropanol. Avantageusement, le solvant protique est l'eau.

Dans le procédé selon l'invention, on fait réagir une solution d'un complexe à transfert de charge de formule (II) préalablement formé, avec un sel d'élément alcalino-terreux. L'anion du complexe à transfert de charges F⁻ va réagir, en solution, avec les ions de l'alcalino-terreux sélectionné du type AT²⁺ (où « AT » symbolise un atome d'élément alcalino-terreux).

Le complexe de transfert de charge est obtenu par la réaction d'une source d'ions fluorure F⁻ telle que de l'acide fluorhydrique avec un amide de formule II présent en excès dans le milieu réactionnel. Avantageusement, le ratio molaire amide de formule II / F⁻ est supérieur ou égal à 2, de préférence compris entre 2 et 40, et plus préférentiellement entre 5 et 12, et encore plus préférentiellement entre 7,5 et 9,5 ; cela minimise la consommation de solvant. L'amide de formule II peut avantageusement jouer le rôle à la fois de réactif et de solvant. Etant donné que l'amide II peut jouer le rôle de solvant et que la quantité d'ions fluorure F⁻ dépend directement de la quantité de sel d'élément alcalino-terreux, le ratio molaire amide de formule II / F⁻ est affecté lorsque la concentration en sel d'élément alcalino-terreux dans le milieu de synthèse est modifiée. Lorsque la synthèse est effectuée à une concentration plus élevée en sel d'élément alcalino-terreux dans le milieu réactionnel, le ratio molaire amide de formule II / F⁻ diminue. Cependant, pour des ratios molaires amide de formule II / F⁻ inférieurs à 2, le sel d'élément alcalino-terreux, n'est pas soluble dans le milieu réactionnel.

De même, une synthèse dans des conditions de dilution du sel d'élément alcalino-terreux plus importantes induit une augmentation du ratio molaire amide de formule II / F⁻. Le complexe de transfert de charge peut être obtenu à des ratios molaires amide de formule Il / F⁻ supérieurs à 40. Cependant, plus le milieu réactionnel contiendra d'amide de formule II, plus le milieu réactionnel sera dilué, entrainant une consommation inutile d'amide de formule II et donc un surcoût inutile.

La réaction entre le sel d'élément alcalino-terreux préalablement solubilisé dans le solvant 1 et le complexe de transfert de charge est effectuée de manière à obtenir un ratio molaire AT/F⁻ inférieur à 1, et de préférence de l'ordre de 0,7. Avantageusement, l'ion fluorure est le réactif limitant de cette réaction. Pour un ratio AT/F⁻ constant, l'augmentation de la concentration en sel d'élément alcalino-terreux dans le milieu réactionnel conduit à une légère augmentation de la taille des nanoparticules obtenues (dans un exemple, la taille moyenne passe d'environ 8 nm à environ 9 nm (voir ci-dessous, références 978a et b)).

Le ratio molaire « solvant 1/ amide de formule II » est compris entre 0,1 et 5, de préférence entre 0,1 et 3, et encore plus préférentiellement entre 0,13 et 1 (une bonne valeur est comprise entre 0,17 et 0,61). De manière surprenante, la demanderesse a constaté que la diminution du ratio solvant 1 / amide de formule II conduit à l'accroissement de la taille des nanoparticules de fluorure d'élément alcalino-terreux.

Dans un premier mode de réalisation du procédé selon l'invention, le milieu réactionnel est ensuite introduit dans un autoclave muni d'un moyen de mesure de la température et d'un système d'agitation. L'autoclave est ensuite hermétiquement fermé et disposé dans un manteau chauffant. On entend ici par autoclave un récipient hermétiquement fermé, c'est-à-dire un récipient qui ne laisse pas échapper une surpression qui se crée lors de l'échauffement d'un volume de gaz ou de liquide contenu dans ledit récipient, même au-delà du point d'ébullition normal dudit liquide. Ce mode de réalisation est appelé ici « synthèse en autoclave ».

Lorsque la température de l'autoclave atteint la température de consigne comprise entre 20 et 200°C, de préférence entre 70 et 190°C, et avantageusement à environ 170°C, le mélange est maintenu à cette température de consigne pendant un temps t sous agitation.

Avantageusement, le mélange est maintenu dans l'autoclave à la température de consigne (170°C) pendant un temps compris entre 1 minute et 6 heures, de préférence entre 30 minutes et 2 heures, et encore plus préférentiellement pendant environ 1 heure.

Dans un deuxième mode de réalisation du procédé selon l'invention, la réaction est effectuée à pression atmosphérique et la température du mélange réactionnel est fixée par la température d'ébullition du solvant ou du mélange de solvants employé. Ce mode de réalisation est appelé ici « synthèse en ballon ». Par rapport à la synthèse en ballon, une synthèse en autoclave présente deux paramètres ajustables : la pression et la température. En effet, la synthèse des nanoparticules de CaF₂ en autoclave a été effectuée, par exemple, dans un mélange Méthanol / N-Méthyl-2-pyrrolidone à 170°C à la pression autogène correspondante (pression en fin de réaction : 10-12 bars). Les inventeurs ont constaté que pour les fluorures de calcium et de magnésium, les deux modes de réalisation peuvent conduire à des produits comparables.

Dans un troisième mode de réalisation, très préféré, on travaille à une température inférieure à la température d'ébullition du solvant le plus volatil, soit inférieure à 80°C si le mélange réactionnel comporte du méthanol. Dans ce cas, une température comprise entre 20°C et 70°C convient, mais on préfère entre 30°C et 60°C, et de préférence entre 45°C et 55°C. En effet, les nanoparticules obtenues après 1h de chauffage à 50°C, après purification, sont plus stables dans l'éthanol que les nanoparticules obtenues après 1h d'agitation à température ambiante, toutes choses égales par ailleurs. Dans ce mode de réalisation, les différences de résultat entre l'utilisation d'un ballon (ouvert) et celle d'un autoclave (fermé) sont faibles voire négligeables, et on préfère l'approche qui est industriellement la plus simple, à savoir celle basée sur l'utilisation d'un ballon (avec condenseur).

Les nanoparticules de fluorure de calcium et de magnésium élaborées par le procédé selon l'invention ont été analysées par diffraction de Rayons X. Une simulation du diagramme de diffraction des nanoparticules par la méthode de Rietveld a été effectuée ; elle permet de déterminer les paramètres de maille et démontre que les nanoparticules de fluorure sont parfaitement cristallisées et exemptes d'impuretés.

Ainsi avantageusement, afin d'obtenir des nanoparticules de fluorure d'élément alcalino-terreux cristallisées, les conditions de synthèses sont optimisées : la température de consigne de l'autoclave contenant le milieu réactionnel est fixée à environ 170°C et le mélange réactionnel est maintenu à cette température de consigne pendant environ 6 heures sous agitation. L'autoclave est ensuite refroidi (vitesse maximale préférée : 10°C/min) avant ouverture et extraction du milieu réactionnel.

Qu'il s'agisse de la synthèse en autoclave ou en ballon, le milieu réactionnel est ensuite introduit dans une solution d'acétone induisant la formation immédiate d'un précipité des nanoparticules de fluorure d'élément alcalino-terreux. De préférence, le solvant utilisé pour précipiter les nanoparticules de fluorure d'élément alcalino-terreux est un solvant non toxique.

Après quelques heures (typiquement 6 heures), le solide a précipité et le surnageant est ensuite éliminé par tout moyen approprié. La suspension résultante est centrifugée. Le culot obtenu est ensuite redispersé dans du méthanol. De préférence, le solvant utilisé pour redisperser le culot est un solvant non toxique tel que l'eau.

La solution obtenue est ensuite soumise à des cycles de centrifugation / redispersion de manière à purifier les nanoparticules de fluorure d'élément alcalino-terreux. Au moins un cycle, de préférence deux cycles de centrifugation / redispersion est (sont) effectué(s) à des accélérations centrifuges typiquement comprises entre 500 g et 15 000 g pendant 5 à 30 minutes. Ce(s) cycle(s) de centrifugation / redispersion permette(nt) de séparer les fluorures d'élément alcalino-terreux des composés n'ayant pas réagi et des sous-produits de réaction présents dans les surnageants.

La redispersion est effectuée en introduisant une certaine quantité (4 mL de méthanol/ g de ATF₂) d'un solvant sur le culot et par l'emploi de tout moyen de dispersion tel que le vortex ou un bain à ultrasons.

Après ces cycles de centrifugation / redispersion, le culot peut être redispersé dans l'eau ou un autre solvant, et la suspension colloïdale obtenue peut être soumise aux ultrasons pendant 1 à 2 min par l'emploi d'une sonde à ultrasons (Hielscher, UP400S).

Dans un mode de réalisation particulier, afin de purifier ces nanoparticules de fluorure d'élément alcalino-terreux, la suspension colloïdale précédente est introduite de nouveau dans une solution d'acétone induisant la formation d'un précipité blanc qui subira de nouveau des cycles de centrifugation / redispersion.

Ces variantes du procédé de synthèse qui viennent d'être décrites peuvent être mises en œuvre avec ses domaines préférentiels notamment pour la synthèse de nanoparticules de fluorure de calcium, baryum et strontium.

Il faut sélectionner au départ un sel d'élément alcalino-terreux soluble dans le solvant utilisé. Dans de nombreux cas un chlorure ou nitrate peut convenir.

### 2) Fonctionnalisation des nanoparticules de fluorure d'élément alcalino-terreux

L'un des avantages du procédé selon l'invention est qu'il conduit directement à des nanoparticules de fluorure d'élément alcalino-terreux fonctionnalisées, la molécule organique fonctionnalisante est soit l'amide de formule II (utilisé comme solvant), soit son produit de dégradation, si le procédé est conduit à une température suffisante pour dégrader l'amide (cela est démontré dans les exemples ci-dessous). Dans certain cas, l'utilisation visée de la suspension de nanoparticules peut nécessiter une autre fonctionnalisation. Les nanoparticules de fluorure d'élément alcalino-terreux précédemment obtenues peuvent alors être fonctionnalisées avec d'autres molécules organiques, selon des méthodes connues de l'homme du métier. La fonctionnalisation consiste à greffer à la surface des nanoparticules une molécule présentant une structure du type A- Sp-Z dans laquelle :
- A est une fonction chimique assurant l'accrochage de la molécule à la surface,
- Sp est un groupe espaceur qui participe à la balance hydrophile / hydrophobe et à la dispersion des particules,
- Z une fonction chimique, réactive ou non, qui participe à la balance hydrophile / hydrophobe, à la dispersion des particules et éventuellement à leur interaction avec leur milieu de dispersion (insertion covalente dans un réseau polymère).

Comme groupe A, une fonction complexante des cations de surface des nanoparticules peut être utilisée (telle que : phosphate, phosphonate, carboxylate, dithiophosphate, dithiophosphonate). La force de complexation de ce groupement peut être renforcée par l'utilisation de fonctions multiples (telles que : polyacide, polyphosphate). Par exemple, une fonctionnalisation de la surface du fluorure d'élément alcalino-terreux obtenu peut être réalisée avec une molécule organique porteuse d'une fonction biphosphonate.

Comme groupe espaceur Sp, on peut utiliser n'importe quel espaceur connu de l'homme du métier, par exemple une chaîne alkyl linéaire ou branchée ou un groupe aromatique ou une combinaison de ces groupes, pouvant être interrompus par un ou plusieurs hétéro atomes choisis parmi O, N, S, P.

Comme fonction chimique Z on peut utiliser n'importe quelle fonction chimique connue de l'homme de l'art telle que les fonctions méthacrylate, acrylate, glycidyl, alcool, méthoxy. Les fonctions alcool et méthoxy sont préférées.

D'autres molécules fonctionnalisantes A-Sp-Z peuvent par exemple être choisies parmi le :
- sel de sodium du methoxy-PEG-C6-phosphonate (aussi connu sous le nom P-7,10,13,16-tetraoxaheptadec-1-yl-phosphonic acid, sodium salt),
- sel de sodium de l'hydroxy-PEG-C6-phosphonate (aussi connu sous le nom P-[6-[2-[2-(2-hydroxyethoxy)ethoxy]ethoxy]hexyl]phosphonic acid, sodium salt),
- sel de sodium de 2-[2-(2-Methoxyethoxy)ethoxy]acetic acid.

Selon un mode de réalisation, une suspension colloïdale de fluorure d'élément alcalino-terreux ATF₂ est ajoutée à une solution d'A-Sp-Z de manière à obtenir un ratio molaire ATF₂/A-Sp-Z compris entre 1 et 100, de préférence entre 5 et 50, et plus préférentiellement entre 10 et 30. Au-delà d'un ratio molaire ATF₂/A-Sp-Z de 50, la molécule A-Sp-Z risque de ne pas être introduite en quantité suffisante pour assurer la stabilité des particules fonctionnalisées ; cela dépend aussi de la taille des particules. Pour un ratio molaire ATF₂/A-Sp-Z inférieur à 5, la fonctionnalisation des nanoparticules de ATF₂ par la molécule A-Sp-Z risque d'induire un encombrement stérique tel, que les particules de fluorure d'élément alcalino-terreux ne peuvent pas être plus fonctionnalisées ; cela dépend aussi de la taille des particules. L'utilisation d'une quantité d'A-Sp-Z supérieure lors de la fonctionnalisation entrainerait une consommation inutile d'A-Sp-Z.

Une suspension colloïdale de fluorure d'élément alcalino-terreux à une concentration massique comprise entre 1% et 30%, de préférence entre 5% et 15%, et encore plus préférentiellement à 5% est utilisée pour réaliser la fonctionnalisation des particules de fluorure d'élément alcalino-terreux. A forte concentration, il peut y avoir un risque de pontage et un manque d'accessibilité de la surface à fonctionnaliser (risque de précipitation de particules non ou mal fonctionnalisées). De préférence, les fluorures d'élément alcalino-terreux sont dispersés dans un solvant non toxique, tel que l'eau ou l'éthanol.

Cette réaction peut être réalisée dans tous solvants appropriés, de préférence dans des solvants non-toxiques, permettant de solubiliser la molécule A-Sp-Z.

En fonction de la molécule A-Sp-Z les conditions de fonctionnalisation peuvent être optimisées (température, durée, solvants...). Après avoir ajouté une solution d'A-Sp-Z à une suspension colloïdale de fluorure d'élément alcalino-terreux, le milieu réactionnel est laissé sous agitation pendant quelques heures, de manière à ce qu'au moins une partie, de préférence la totalité des molécules A-Sp-Z puisse être greffée à la surface des particules de fluorure d'élément alcalino-terreux. La fonctionnalisation peut être réalisée sous chauffage, de préférence à une température comprise entre 40°C et 70°C. La température du milieu réactionnel doit être adaptée au choix de la molécule fonctionnalisante.

Avantageusement, les nanoparticules ainsi fonctionnalisées sont ensuite purifiées directement ou après introduction dans un solvant entraînant leur précipitation (par exemple l'acétone ou l'éther). La purification se fait par des cycles de centrifugations et redispersions successives et /ou par filtration tangentielle.

Dans un mode de réalisation particulier, la suspension colloïdale de particules de fluorure d'alcalino-terreux fonctionnalisées est centrifugée pendant un temps t compris entre 5 et 30 minutes, entre 648 et 16 211 g, de manière à séparer les particules fonctionnalisées des molécules n'ayant pas réagi présentes dans le surnageant. Le surplus de molécules A-Sp-Z, n'ayant pas réagi et toujours présentes dans le surnageant, est désigné ci-après « molécules fonctionnalisantes libres ». Après centrifugation, le surnageant est éliminé. Le culot comprenant les particules fonctionnalisées est redispersé dans le solvant. Cette redispersion peut être effectuée par tout moyen, notamment par l'emploi d'un bain à ultrasons ou l'emploi d'une sonde à ultrasons (Hielscher, UP400S), ou encore sous agitation magnétique et/ou manuelle. Plusieurs moyens de redispersion peuvent être combinés pour permettre une bonne redispersion des particules dans le solvant.

Plusieurs cycles de centrifugations et redispersions successives peuvent être effectués de manière à éliminer les molécules n'ayant pas réagi. De préférence, on réalise au moins un, encore plus préférentiellement au moins deux cycles de centrifugations et redispersions successifs.

La redispersion des particules de fluorure d'élément alcalino-terreux fonctionnalisées peut être réalisée dans un solvant non toxique, notamment dans l'eau.

La redispersion peut être réalisée en plusieurs étapes. Après centrifugation, le surnageant est extrait. Le solvant ajouté sur le culot permet de redisperser au moins une partie du culot. La solution colloïdale issue de la mise en solution d'une partie du culot est extraite et du solvant est à nouveau ajouté sur le culot restant. Cette opération est alors répétée jusqu'à la redispersion complète du culot. Les fractions de la solution colloïdale récupérées sont ensuite rassemblées.

Après redispersion des particules de fluorure d'élément alcalino-terreux fonctionnalisées, la suspension peut être reconcentrée jusqu'à atteindre l'extrait sec souhaité, par tout moyen approprié, et notamment à l'aide d'un évaporateur rotatif.

Ce procédé de fonctionnalisation qui vient d'être décrit peut être mis en œuvre avec ses domaines préférentiels notamment pour la synthèse de nanoparticules de fluorure de calcium.

Il est entendu que dans le cadre de la présente invention, le procédé de purification et redispersion qui vient d'être décrit peut aussi être effectué à l'issue de l'étape de synthèse décrit ci-dessus sous point 1).

### 3) Fabrication d'un produit pour traitement prophylactique ou curatif des dents

Les inventeurs ont trouvé que la suspension selon l'invention peut être utilisée pour la fabrication d'un produit pour le traitement prophylactique ou curatif des dents. L'utilisation d'une poudre de CaF₂ nanocristallin préparée par une technique de séchage par pulvérisation pour la fabrication d'un produit pour le traitement prophylactique ou curatif des dents a été décrite dans la littérature (voir L. Sun et L. Chow, « Preparation and properties of nano-sized calcium fluoride for dental applications » , paru en 2008 dans la revue Dental Materials 24, p. 111-116 ; voir H. Xu, J. Moreau et a., « Strength and fluoride release characteristics of a calcium fluoride based dental nanocomposite », paru en 2008 dans la revue Biomaterials 29, p. 4261-4267). Dans le procédé décrit dans ces deux publications, les particules de CaF₂ ont été piégées par un séparateur électrostatique après leur précipitation par la réaction Ca(OH)₂ + NH₄F → CaF₂↓ + NH₃↑+ H₂O↑. Dans la première de ces deux publications, les particules de CaF₂ avaient une taille moyenne d'environ 41 nm ; dans la seconde, la taille des particules était comprise entre 15 nm et 335 nm avec une valeur à D₅₀ de 56 nm. Un composite pour le comblement dentaire comprenant la poudre de CaF₂, des whiskers de nitrure de silicium, des nanoparticules de CaHPO₄ et une base de résine à deux composants (comprenant Bis-GMA et TEGDMA à parts égales avec soit un initiateur soit un accélérateur) est décrit dans la publication citée de Xu et al. L'inconvénient de cette méthode réside, d'une part, dans la nécessité de manipuler et disperser des poudres nanométriques de CaF₂, et, d'autre part, dans la mauvaise définition de ces poudres de CaF₂ dont la taille varie dans une fourchette très large.

Selon la présente invention, on utilise directement la suspension de nanoparticules fonctionnalisées de CaF₂ obtenue par le procédé selon l'invention décrit ci-dessus pour la fabrication d'un produit pour le traitement prophylactique ou curatif des dents. Cette suspension peut notamment être utilisée directement dans la fabrication d'un matériau de comblement dentaire, tel qu'une résine, en choisissant dans la préparation de la suspension un solvant de redispersion approprié ; à titre d'exemple, ce solvant peut être choisi de manière à convenir pour le procédé de préparation d'une résine dentaire. Selon l'invention, ce solvant de redispersion peut être un monomère utilisé pour la fabrication d'une résine dentaire, tel que le triethylene glycol dimethacrylate (TEGDMA) ou le bisphenol glycidyl diméthylacrylate (Bis-GMA), ce qui permet d'obtenir des résines telles que celle décrite dans la publication citée de Xu et al. ; d'autres charges peuvent être utilisées. C'est en particulier pour cette application dans la fabrication de matériaux de comblement dentaire que la possibilité du procédé selon l'invention de générer des suspension à fort extrait sec est particulièrement intéressante.

Un autre produit pour le traitement prophylactique ou curatif des dents qui peut être fabriqué avec les suspensions selon l'invention sont les vernis dentaires. L'utilisation de vernis fluorés en odontologie est connue en tant que telle (voir par exemple la thèse de doctorat de Marion Le Nevet, « Les vernis fluorés en odontologie pédiatrique », présentée en 2012 à l'Université de Nantes (UFR d'Odontologie, n° 054) ; voir également la publication « Fluoride varnishes and caries incidence decrease in preschool children : a systematic review » par D.M. Calvalho et al., parue en 2010 dans Rev. Bras. Epidemiol, 13(1), p. 1-11 ; voir également l'article « Substantive Fluoride Release from a New Fluoride Varnish Coating Containing CXP™" par J.L. Milburn et al, paru dans la revue Dentistry, 5(12) en 2015). Cependant, les produits connus ne comportent pas de CaF₂ mais des formes plus solubles de fluorure (NaF le plus souvent). C'est la forme nanocristalline du CaF₂ qui, selon l'invention, assure une solubilité d'équilibre suffisante pour assurer une concentration suffisante d'ions fluorure dans la salive.

Dans le cadre de la présente invention, on peut utiliser un vernis à base de résine naturelle (telle que : colophane (rosin), gomme laque, mastic, ou des mélanges de ces résines naturelles) et/ou synthétique, éventuellement dilué dans un solvant acceptable d'un point de vue pharmaceutique tel que l'éthanol ; on peut ajouter des promoteurs d'adhésion, typiquement des dérivés organiques de l'acide phosphorique.

La suspension selon l'invention peut également être utilisée dans la formulation de produits de rinçage buccal, de pâtes dentifrices, ou de gels dentaires pour application topique, en tant que source d'ions de fluorure susceptibles de s'incorporer dans la maille apatitique.

Selon l'invention, l'utilisation directe d'une suspension de nanoparticules présente de nombreux avantages. En particulier, elle évite la manipulation de nanopoudres sèches et assure l'approvisionnement de suspensions stables, reproductibles et de composition contrôlée, sans agglomérat.

### Exemples

L'invention est illustrée ci-dessous par des exemples qui, cependant, ne limitent pas l'invention. Ces exemples portent sur des suspensions colloïdales de nanoparticules de fluorure d'élément alcalino-terreux ATF₂, le procédé de synthèse de nanoparticules de ATF₂ et la fonctionnalisation de ces nanoparticules.

### Exemple 1-1 : Synthèse de nanoparticules de fluorure de calcium fonctionnalisées en autoclave (solvant NMP)

100 g (0,6802 mol) de CaCl₂, 2H₂O (masse molaire 147,01 g/mol) ont été solubilisés dans 685 mL de méthanol. Par ailleurs, on a introduit dans un récipient en Teflon™ 41,5 g de HF d'une densité de 1,15 g/cm³ (36,1 mL, correspondant à 1,0162 mol, masse molaire 20,01 g/mol, 48% massique dans l'eau) et ajouté 2,66 L de N-Méthyl-2-pyrrolidone (abrégé « NMP », n° CAS 872-50-4). On a ensuite ajouté à cette solution de HF et NMP sous agitation magnétique la phase liquide de CaCl₂ dans le méthanol. Le récipient en Teflon™ a été placé dans un autoclave. L'autoclave a été fermé et chauffé pendant 6 heures à 170°C sous agitation. A la fin de cette réaction on a laissé refroidir et l'autoclave a été ouvert. La phase liquide a été versée dans 4 L d'acétone pour précipitation ; on a ensuite centrifugé pendant 20 minutes à 10 000 g et éliminé le surnageant. Le culot a été redispersé dans 240 mL de méthanol et a été lavé une seconde foi. On a ensuite ajouté 40 mL d'eau pour redisperser et obtenir une solution colloïdale. Cette dernière contient 39,7 g (0,5081 mol) de CaF₂ (masse molaire 78,07 g/mol).

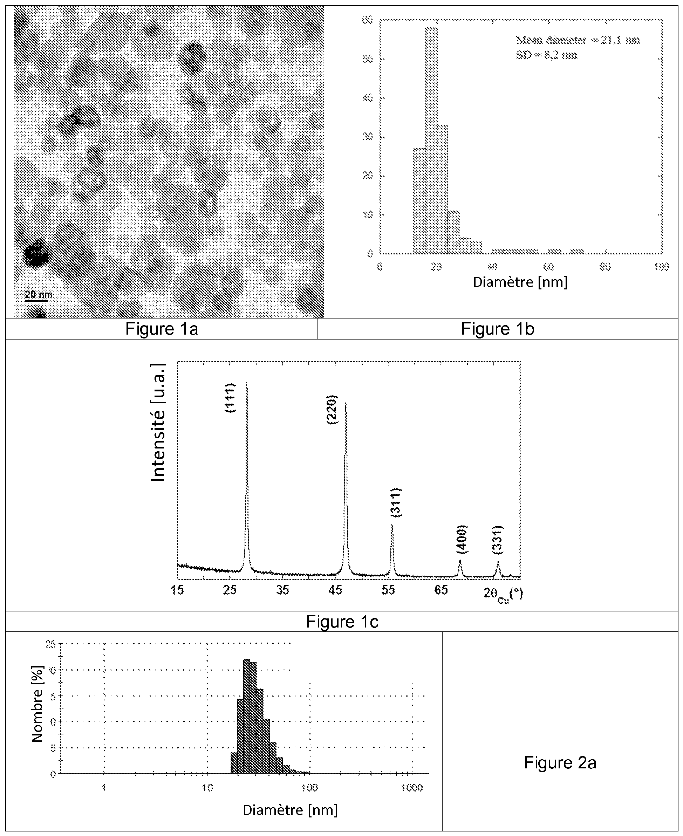

La figure 1a montre une image typique obtenue par microscopie électronique en transmission des nanoparticules synthétisées. La figure 1b montre la distribution en taille des nanoparticules de laquelle on déduit le taille moyenne des grains avec son écart-type : 21 ,1 nm ± 8,2 nm. La figure 1c montre un diffractogramme des Rayons X de ces nanoparticules: ces nanoparticules sont bien cristallisées (système cristallin cubique, groupe d'espace Fm-3m). A ce stade, la solution peut être purifiée pour obtenir une solution aqueuse ou transférée dans l'éthanol.
- Variante « solution aqueuse » : Le colloïde aqueux a été dilué puis dialysé, et on a récupéré une solution à 18,7 % massiques de CaF₂.
- Variante « éthanol » : La solution aqueuse a été précipitée de l'acétone puis centrifugée pendant 5 minutes à 6500g. On a ajouté 200 mL d'éthanol pour redisperser les nanoparticules. La solution colloïdale obtenue a été reconcentrée à l'évaporateur rotatif jusqu'à un extrait sec de 10,6% en masse.

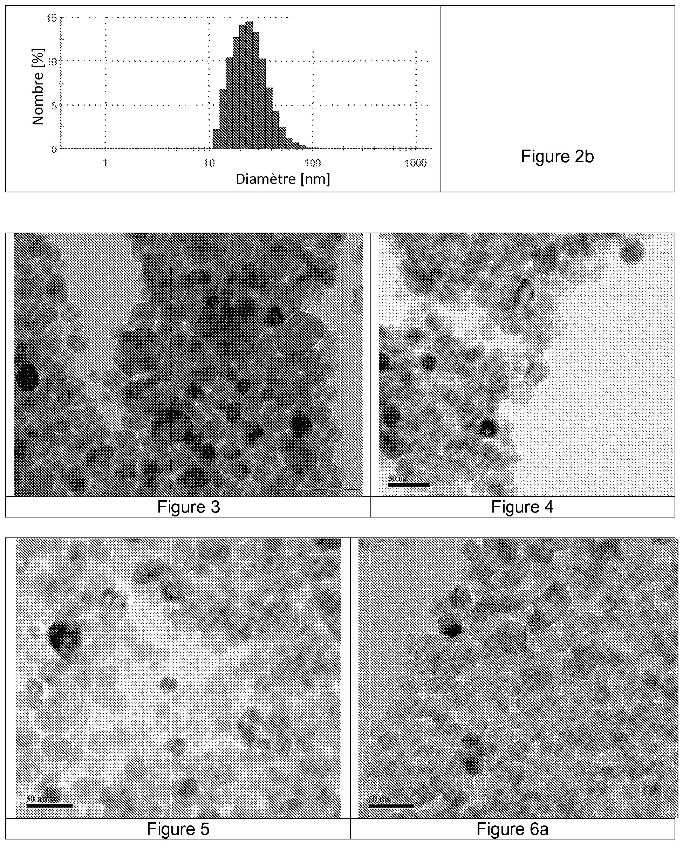

Les figures 2a et 2b montrent le diamètre moyen des particules obtenu par diffusion dynamique de la lumière (DLS - Dynamic Light Scattering) respectivement dans l'eau (figure 2a) et dans l'éthanol (figure 2b). On trouve les valeurs suivantes ;

Diamètre hydrodynamique dans l'eau = 30,1 nm, indice de polydispersité = 0,195. Diamètre hydrodynamique dans l'éthanol = 26,1 nm, indice de polydispersité = 0,246.

On note que les valeurs de diamètre obtenues par DLS sont supérieures à celles obtenues par microscopie, car la technique DLS détermine un diamètre hydrodynamique qui prend en compte les sphères de solvatation de la particule. L'eau étant plus polaire que l'éthanol, les couches de solvatation sont plus épaisses pour l'eau que pour l'éthanol. La valeur obtenue par DLS prend également en compte la couche de fonctionnalisation.

### Exemple 1-2 : Synthèse de nanoparticules de fluorure de calcium fonctionnalisées en autoclave (solvant NMP)

On a répété l'exemple 1-1 avec les quantités suivantes :
5 g (0,03401 mol) de CaCl₂, 2H₂O dans 34,3 mL de méthanol ; 2,075 g de HF (1,8 mL, correspondant à 0,0508 mol), 133 mL de NMP. Chauffage en autoclave 6 heures à 170°C sous agitation. La phase liquide a été versé dans 200 mL d'acétone pour précipitation ; centrifugation 20 minutes à 10 000 g, redispersion du culot dans 40 mL de méthanol. Après un deuxième lavage ajout de 10 mL d'eau pour redisperser et obtenir une solution colloïdale de CaF₂. La taille des particules visible sur la figure 3 (micrographie de microscopie électronique à transmission) était de l'ordre de 20 à 50 nm. On a obtenu un diagramme de diffraction X très similaire à celui de la figure 1c.

### Exemple 2 : Synthèse de nanoparticules de fluorure de calcium en autoclave (solvant : pyrolinidone)

80 g (0,5442 mol) de CaCl₂, 2H₂O (masse molaire 147,01 g/mol) ont été solubilisés dans 535 mL de méthanol. Par ailleurs, on a introduit dans un récipient en Teflon™ 33,2 g de HF d'une densité de 1,15 g/cm³ (28,9 mL, correspondant à 0,8130 mol, masse molaire 20,01 g/mol) et ajouté 532 mL de 2-pyrrolidinone. On a ensuite ajouté à cette solution de HF et pyrrolidinone sous agitation magnétique la phase liquide de CaCl₂ dans le méthanol. Le récipient en Teflon™ a été placé dans un autoclave. L'autoclave a été fermé et chauffé pendant 1 heure à 170°C sous agitation. A la fin de cette réaction, on a laissé refroidir et on a ouvert l'autoclave. La phase liquide a été versée dans 800 mL d'acétone pour précipitation ; on a ensuite centrifugé pendant 20 minutes à 10 000 g et éliminé le surnageant. Le culot a été redispersé dans 150 mL de méthanol et a été lavé une seconde fois. On a ensuite ajouté 40 mL d'eau pour redisperser et obtenir une solution colloïdale. Le produit présentait une morphologie et cristallinité similaires à celles du produit obtenu à l'exemple 1.

### Exemple 3 : Synthèse de nanoparticules de fluorure de calcium fonctionnalisées en autoclave (solvant ; NMP / éthylène glycol)

9,87 g (0,06725 mol) de CaCl₂, 2H₂O ont été solubilisés dans un mélange de 10 mL d'éthylène glycol et 50 mL de NMP. Par ailleurs, on a introduit dans un récipient en Teflon™ 4,23 g de HF d'une densité de 1,15 g/cm³ (3,67 mL, correspondant à 0,1035 mol) et ajouté 85 mL de NMP. On a ensuite ajouté à cette solution de HF et NMP sous agitation magnétique la phase liquide comportant le CaCl₂. Le récipient en Teflon™ a été placé dans un autoclave. L'autoclave a été fermé et chauffé pendant 6 heures à 170°C sous agitation. A la fin de cette réaction, on a laissé refroidir et on a ouvert l'autoclave. La phase liquide a été versée dans 200 mL d'acétone pour précipitation ; on a ensuite centrifugé pendant 20 minutes à 10 000 g et éliminé le surnageant. Le culot a été redispersé dans 40 mL de méthanol et a été lavé une seconde fois. On a ensuite ajouté 10 mL d'eau pour redisperser et obtenir une solution colloïdale.

Le produit présentait une morphologie et cristallinité similaires à celles du produit obtenu à l'exemple 1. La taille des particules visibles sur la figure 4 était de l'ordre de 20 nm à 30 nm.

### Exemple 4 : Synthèse de nanoparticules de fluorure de calcium fonctionnalisées en ballon (solvant : NMP)

5 g (0,03401 mol) de CaCl₂, 2H₂O ont été solubilisés dans 34,3 mL de méthanol. Par ailleurs on a introduit 133 mL de NMP dans un ballon d'un volume de 250 mL, et on a ajouté 2,075 g de HF (1,8 mL, correspondant à 0,0508 mol). On a ensuite ajouté la solution de CaCl₂ dans le ballon, et on a gardé le mélange pendant 15 h à reflux (température du bain : 170°C) sous agitation. La phase liquide a ensuite été versée dans 200 mL d'acétone pour précipitation ; on a centrifugé pendant 20 minutes à 10 000 g, écarté le surnageant, et redispersé le culot dans 50 mL de méthanol. Après un deuxième lavage au méthanol, on a ajouté 10 mL d'eau pour redisperser et obtenir une solution colloïdale de CaF₂. La taille des particules visible sur la figure 5 (micrographie de microscopie électronique à transmission) était de l'ordre de 20 à 25 nm. On a obtenu un diagramme de diffraction X très similaire à celui de la figure 1c.

### Exemple 5 : Synthèse de nanoparticules de fluorure de magnésium fonctionnalisées en autoclave (solvant : NMP)

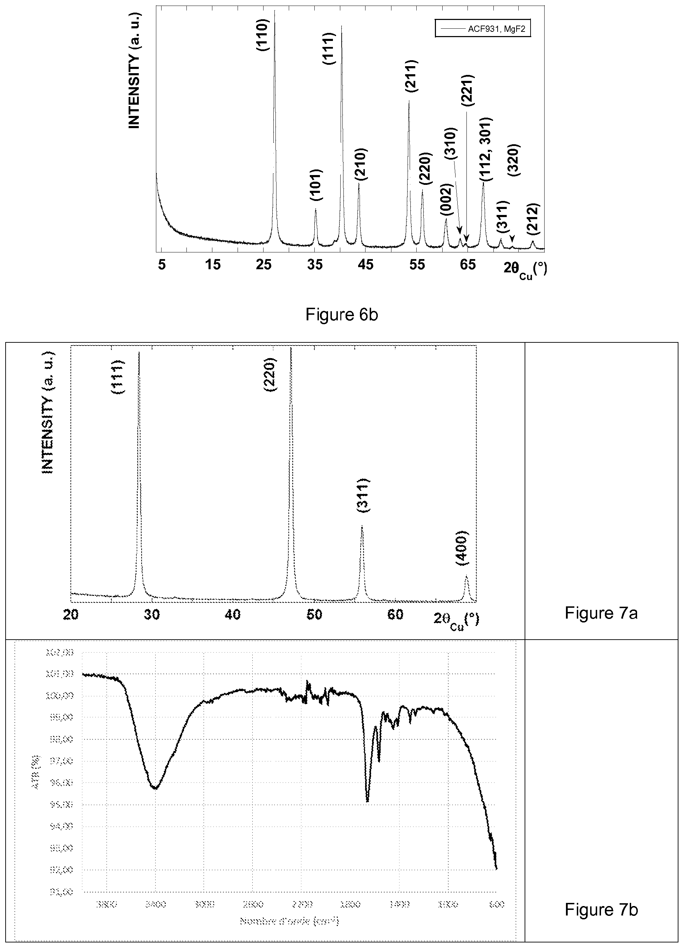

6 g (0,03401 mol) de MgCl₂, 4,5H₂O (masse molaire 176,29 g/mol) ont été solubilisés dans 34,3 mL de méthanol. Par ailleurs, on a introduit dans un récipient en Teflon™ 2,075 g de HF (1,8 mL, correspondant à 0,0508 mol) et ajouté 133 mL de NMP. On a ensuite ajouté à cette solution de HF et NMP sous agitation magnétique la phase liquide de MgCl₂ dans le méthanol. Le récipient en Teflon™ a été placé dans un autoclave. L'autoclave a été fermé et chauffé pendant 6 heures à 170°C sous agitation. A la fin de cette réaction, on a laissé refroidir et on a ouvert l'autoclave. La phase liquide a été versé dans 200 mL d'acétone pour précipitation ; on a ensuite centrifugé pendant 20 minutes à 10 000 g et éliminé le surnageant. Le reste a été redispersé dans 40 mL de méthanol et a été lavé une seconde fois au méthanol. On a ensuite ajouté 10 mL d'eau pour redisperser et obtenir une solution colloïdale de MgF₂. La taille des particules visible sur la figure 6a (micrographie de microscopie électronique à transmission) était de l'ordre de 20 à 30 nm. La figure 6b montre un diffractogramme aux Rayons X de cette poudre : la poudre est bien cristallisée (système cristallin tétragonal, groupe d'espace P42/mmm).

### Exemples 6 à 24 : Diverses synthèses de nanoparticules de fluorure de calcium fonctionnalisées

### Exemples de Groupe I (exemples 6, 9, 10, 11, 12, 13, 14, 15, 16, 18, 21, 23, 24) :

On a solubilisé 2 g (0,0136 mol) de CaCl₂.2H₂O dans 13,7 mL de MeOH et ajouté 722 µL (0,833 g = 0,0203 mol) de HF (concentration 49% en Mol/L) dans 53,2 mL NMP sous agitation magnétique.

### Exemples de Groupe II (exemples 7,8) :

On a solubilisé 5 g (0,03401 mol) de CaCl₂.2H₂O dans 11,5 mL de MeOH (34,3 mL pour l'exemple 8) et ajouté 1,804 mL (2,075 g = 0,0508 mol) de HF (concentration 49%) dans 133 mL NMP (pour l'exemple 8 : 155,8 mL) sous agitation magnétique.

### Exemples de Groupe III (exemples 17,19,20,22) :

On a solubilisé 8 g (0,05442 mol) de CaCl₂.2H₂O dans 13,7 mL de MeOH et ajouté 2,88 mL (3,332 g = 0,08128 mol) de HF (concentration 49%) dans 53,2 mL pyrrolidinone sous agitation magnétique.

Pour tous les exemples 6 à 24, on a ajouté la solution de CaCl₂ sous agitation magnétique à la solution de HF dans l'amide, puis on a traité le mélange réactionnel dans le réacteur indiqué, à la température et pendant la durée indiquées en tableau 1. Pour la synthèse en autoclave, on note que l'autoclave est placé dans une étuve dans laquelle règne la température de consigne indiquée dans le tableau 1, que la durée est comptée à partir du moment où l'enveloppe extérieure de l'autoclave a atteint la température de consigne, et qu'à la fin du temps indiqué, l'autoclave est sorti de l'étuve pour être refroidi rapidement sous courant d'air comprimé.

Puis on a exécuté les étapes suivantes :
- Le mélange réactionnel est versé sur une quantité définie d'acétone (Groupe I : 100 mL ; Groupe II : 180 mL ; Groupe III : 100 mL) pour faire précipiter les particules de CaF₂,
- Le mélange est centrifugé pendant 5 minutes à 8 000 g,
- On ajoute une quantité définie de MeOH sur le culot pour le redisperser, (Groupe I : 10 mL ; Groupe II : 60 mL ; Groupe III : 60 mL) et on centrifuge pendant 5 minutes à 6000 g, sachant que cette étape d'ajout de MeOH et de cenrifugation est répétée une fois ;
- On ajoute une quantité définie d'eau pour redisperser le culot ; on obtient ainsi une solution colloïdale (Groupe I : 5 mL ; Groupe II : 10 mL ; Groupe III : 20 mL).

**Tableau 1 : Conditions de préparation pour les exemples 6 à 24**

| Ex | Echantillon | Réacteur | Amide | Solvant de dissolution de CaCl₂ | Ratio volumique amide/ Solvant dissolution | Température de traitement | Temps de réaction |
|---|---|---|---|---|---|---|---|
| 6 | ACF 959 | autoclave | NMP | MeOH | 3,9 | 170°C | 1h |
| 7 | ACF 960 | ballon | NMP | MeOH | 3,9 | 170°C | 1h |
| 8 | ACF 961 | ballon | NMP | MeOH | 13,5 | 170°C | 1h |
| 9 | ACF 964a | autoclave | NMP | MeOH | 3,9 | 170°C | 0 min |
| 10 | ACF 964b | autoclave | NMP | MeOH | 3,9 | 170°C | 30 min |
| 11 | ACF 965a | autoclave | NMP | MeOH | 3,9 | 50°C | 1h |
| 12 | ACF 965b | autoclave | NMP | MeOH | 3,9 | 100°C | 1h |
| 13 | ACF 966a | ballon | P | MeOH | 3,9 | 170°C | 1h |
| 14 | ACF 966b | autoclave | P | MeOH | 3,9 | 170°C | 1h |
| 15 | ACF 969 | ballon | NMP | MeOH | 3,9 | 80°C | 1h |
| 16 | ACF 978a | autoclave | P | MeOH | 3,9 | 50°C | 1h |
| 17 | ACF 978b | autoclave | P | MeOH | 3,9 | 50°C | 1h |
| 18 | ACF 978c | bécher | P | MeOH | 3,9 | RT | 1h |
| 19 | ACF 978d | bécher | P | MeOH | 3,9 | RT | 1h |
| 20 | ACF 979a | autoclave | NMP | MeOH | 3,9 | 50°C | 1h |
| 21 | ACF 979b | autoclave | NMP | Eau | 3,9 | 50°C | 1h |
| 22 | ACF 979c | bécher | NMP | MeOH | 3,9 | RT | 1h |
| 23 | ACF 980a | ballon | NMP | Eau | 3,9 | 80°C | 1h |
| 24 | ACF 980b | bécher | NMP | Eau | 3,9 | RT | 1h |
| Dans la colonne « Amide », la lettre « P » signifie « pyrrolidinone ». | | | | | | | |

**Tableau 2 : Propriétés des produits obtenus par les exemples 6 à 24**

| Ex | Echantillon | m(CaF₂) attendu pour un autoclave de 100g | Diamètre hydrodynamique (DLS) | Diamètre moyen (MET) | Déviation standard (MET) | Figures |
|---|---|---|---|---|---|---|
| 6 | ACF 959 | 0,793g | 24,1 nm | | | 7a, 7b |
| 7 | ACF 960 | 0,793g | | 48,7 nm | 19,4 nm | 8a, 8b, 8c, 8d |
| 8 | ACF 961 | 0,793g | 69,0 nm | | | |
| 9 | ACF 964a | 0,793g | 30,4 nm | 23,6 nm | 6,2 nm | 9a, 9b, 9c |
| 10 | ACF 964b | 0,793g | 33,5 nm | | | |
| 11 | ACF 965a | 0,793g | 59,8 nm | 14,5 nm | 2,9 nm | 10a, 10b, 10c |
| 12 | ACF 965b | 0,793g | 45,9 nm | | | |
| 13 | ACF 966a | 0,793g | 38,3 nm | 9,97 nm | 2,1 nm | |
| 14 | ACF 966b | 0,793g | 14,8 nm | 10,0 nm | 2,1 nm | 11a, 11b, 11c |
| 15 | ACF 969 | 0,793g | 24,7 nm | 17,1 nm | 3,3 nm | 12a, 12b, 12c |
| 16 | ACF 978a | 0,793 g | 37,5 nm | 7,7 nm | 1,4 nm | 13a, 13b, 13c, 13d, 13^{e} |
| 17 | ACF 978b | 3,173 g | 38,4 nm | 9,5 nm | 2,0 nm | 14a, 14b, 14c |
| 18 | ACF 978c | 0,793 g | | | | |
| 19 | ACF 978d | 3,173 g | 35,8 nm | 9,7 nm | 2,0 nm | 15a, 15b, 15c |
| 20 | ACF 979a | 3,173 g | 35,6 nm | 12,8 nm | 3,2 nm | 16a, 16b, 16c |
| 21 | ACF 979b | 0,793 g | 93,1 nm | | | |
| 22 | ACF 979c | 3,173 g | 62,8 nm | | | |
| 23 | ACF 980a | 0,793 g | 157,5 nm | 87,2 nm | 22,2 nm | |
| 24 | ACF 980b | 0,793 g | | 81,5 nm | 23,9 nm | |

On note que dans le tableau 2, le diamètre moyen des particules obtenu par DLS prend en compte à la fois la couche de fonctionnalisation et la couche de solvatation, alors que le diamètre moyen obtenu par MET ne prend en compte ni la couche de fonctionnalisation (qui n'est pas visible sous les conditions expérimentales choisies) ni la couche de solvatation (l'observation MET étant effectuée sur un échantillon de poudre sèche).

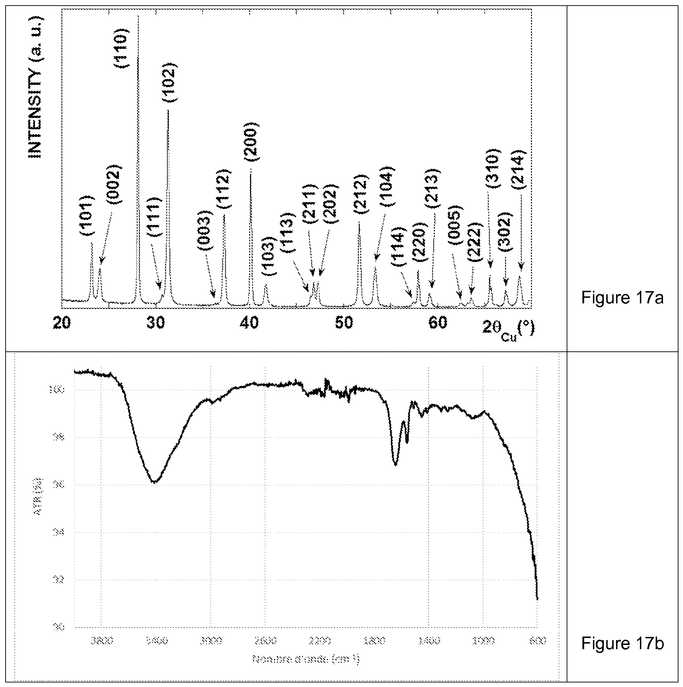

### Exemple 25 : Synthèse de nanoparticules de bromure fluorure de baryum fonctionnalisées en ballon (solvant : NMP) - échantillon ACF 963 (figures 17a, 17b)

9,282 g (0,0312 mol) de BaBr₂ ont été solubilisés dans 46,83 mL de méthanol. Par ailleurs, on a introduit 125 mL de NMP dans un ballon d'un volume de 250 mL, et on a ajouté 1,911 g de HF à 49% en mol/L (1,66 mL, correspondant à 0,0468 mol) sous agitation magnétique. On a ensuite ajouté la solution de BaBr₂ dans le ballon, et on a gardé le mélange pendant 1 h à reflux (température du bain : 170°C) sous agitation. La phase liquide a ensuite été versée dans 200 mL d'acétone pour précipitation ; on a centrifugé pendant 10 minutes à 10 000 g, écarté le surnageant et redispersé le culot dans 60 mL de méthanol. Après un deuxième lavage au méthanol, on a ajouté 30 mL d'eau pour redisperser et obtenir une solution colloïdale de BaBrF. Les nanoparticules ont été caractérisées par diffraction des Rayons X (figure 17a).

### Exemple 26 : Synthèse de nanoparticules de SrFCl + SrF₂ fonctionnalisées en ballon (solvant : NMP) - échantillon ACF 970

3,63 g (0,0136 mol) de SrCl₂.6H₂O ont été solubilisés dans 13,7 mL de méthanol. Par ailleurs on a introduit 53,2 mL de NMP dans un ballon, et on a ajouté 0,83 g de HF à 49% en mol/L (0,722 mL, correspondant à 0,02032 mol). On a ensuite ajouté la solution de SrCl₂ dans le ballon sous agitation magnétique, et on a gardé le mélange pendant 1 h à reflux (température du bain : 170°C) sous agitation. La phase liquide a ensuite été versée dans 180 mL d'acétone pour précipitation ; on a centrifugé pendant 5 minutes à 6 000 g, écarté le surnageant, et redispersé le culot dans 60 mL de méthanol. Après un deuxième lavage au méthanol, on a ajouté 60 mL d'eau pour redisperser et obtenir une solution colloïdale de SrFCl + SrF₂. Les nanoparticules ont été caractérisées par diffraction des Rayons X.

### Exemple 27 : Synthèse de nanoparticules de CaF₂ fonctionnalisées en ballon (solvant : pyrrolidinone)

8 g (0,05442 mol) de CaCl₂.2H₂O ont été solubilisés dans 13,7 mL de méthanol. Par ailleurs on a introduit 4,46 g de triéthylamine trihydrofluoride (Et₃N.3HF, n° CAS 73602-61-6) à 98% (correspondant à 0,02710 mol) dans 53,2 mL de pyrrolidinone dans un ballon sous agitation magnétique. On a ensuite ajouté la solution de CaCl₂ dans le ballon sous agitation magnétique, et on a gardé le mélange pendant 1 h à 50°C sous agitation. Le ballon a ensuite été refroidi à l'air comprimé, et la phase liquide a été versée dans 100 mL d'acétone pour précipitation ; on a centrifugé pendant 10 minutes à 8 000 g, écarté le surnageant, et redispersé le culot dans 60 mL de méthanol. Après un deuxième lavage au méthanol, on a ajouté 20 mL d'eau pour redisperser le culot et obtenir une solution colloïdale de CaF₂. Les nanoparticules ont été caractérisées par diffraction des Rayons X et absorption infrarouge. Le diamètre hydrodynamique était de 23,9 nm.

### Exemple 28 : Fonctionnalisation de CaF₂ par le sel de sodium de l'acide 2-[2-(2-méthoxyéthoxy)éthoxy] acétique (MEEEA)

20 g de colloïde aqueux de CaF₂ à 5% massique (soit l'équivalent de 1 g (0,01281 mol) de CaF₂) ont été pesés dans un ballon de 100 ml. On a ajouté sous forte agitation 1,6 mL d'une solution aqueuse de sel de sodium de MEEEA (0,41 M, pH = 12). Le ballon a été placé pendant 1 h sous agitation à 50° ; la solution est devenue parfaitement translucide. Elle peut être utilisé telle quelle.

## Revendications

1. Procédé de préparation d'une suspension colloïdale de nanoparticules de fluorure d'élément alcalino-terreux fonctionnalisées avec un extrait sec d'au moins 5 % massiques (de préférence d'au moins 10 %, et plus préférentiellement d'au moins 15 %, et encore plus préférentiellement d'au moins 25 %), dans lequel :
(a) on prépare une solution d'un sel de d'élément alcalino-terreux correspondant,
(b) on prépare une solution d'un complexe de transfert de charge de formule (I) 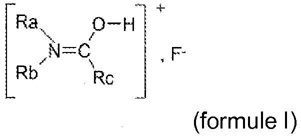 par réaction d'un amide de formule (II) 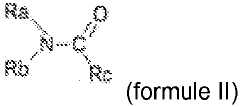 avec une source d'ions fluorure, de préférence de l'acide fluorhydrique, du fluorure de tétrabutylammonium, du trihydrofluorure de triéthylamine ou de l'acide fluorosilicique,
où : Ra et Rb, identiques ou différents, représentent, chacun indépendamment l'un de l'autre, un groupe (C₁-C₆)alkyle, (C₃-C₇)cycloalkyle, phényle ou hétérocycloalkyle de 5 ou 6 chainons, ledit groupe pouvant éventuellement être substitué, ou bien Ra et Rb sont liés entre eux et forment une chaine alkylène, comprenant de 2 à 6 atomes de carbone, éventuellement substituée, et Rc représente un atome d'hydrogène ou un groupe (C₁-C₆)alkyle, (C₃-C₇)cycloalkyle, phényle ou hétérocycloalkyle de 5 ou 6 chainons, ledit groupe pouvant éventuellement être substitué, ou bien Rb et Rc sont liés entre eux et forment une chaine alkylène, comprenant de 2 à 5 atomes de carbone, éventuellement substituée,
(c) on met en contact, dans un solvant ou mélange de solvants, la solution du sel de d'élément alcalino-terreux correspondant obtenue à l'étape (a) et la solution du complexe de transfert de charge de formule (I) obtenue à l'étape (b),
(d) on laisse réagir, éventuellement en chauffant, le mélange obtenu à l'étape (c) de manière à obtenir une suspension colloïdale de nanoparticules de fluorure d'élément alcalino-terreux,
(e) optionnellement, on lave la suspension colloïdale de nanoparticules de fluorure d'élément alcalino-terreux, de préférence par au moins un cycle de centrifugation/redispersion dans un solvant approprié jusqu'à ce que le surnageant soit limpide et incolore, étant entendu que le dernier solvant de redispersion est choisi en fonction du solvant d'utilisation de la suspension colloïdale de nanoparticules de fluorure d'élément alcalino-terreux,
sachant que les étapes (a) et (b) peuvent être inversées.

2. Procédé selon la revendication 1, dans lequel à l'étape b, ladite source d'ions fluorure est une solution d'acide fluorhydrique.

3. Procédé de préparation d'une suspension colloïdale de nanoparticules de fluorure d'élément alcalino-terreux fonctionnalisées dans lequel :
(i) on prépare une suspension colloïdale de nanoparticules de fluorure d'élément alcalino-terreux en utilisant le procédé selon la revendication 1 ou 2,
(ii) on greffe à la surface des nanoparticules de fluorure d'élément alcalino-terreux une molécule présentant une structure du type A-Sp-Z dans laquelle :
∘ A est une fonction assurant l'accrochage de la molécule à la surface,
∘ Sp est un groupe espaceur,
∘ Z est une fonction chimique permettant de modifier le caractère de surface des nanoparticules,
(iii) optionnellement, on lave la suspension colloïdale de nanoparticules de fluorure de l'élément alcalino-terreux fonctionnalisées, de préférence par au moins un cycle de centrifugation/redispersion dans un solvant approprié jusqu'à ce que le surnageant soit exempt de molécules fonctionnalisantes libres, étant entendu que le dernier solvant de redispersion est choisi en fonction du solvant d'utilisation de la suspension colloïdale de nanoparticules de fluorure d'élément alcalino-terreux fonctionnalisées.

4. Procédé selon l'une quelconque des revendications 1 à 3, **caractérisé en ce que** l'élément alcalino-terreux est choisi parmi le magnésium, le calcium, le baryum, le strontium.

5. Procédé selon l'une quelconque des revendications 1 à 4, **caractérisé en ce que** ledit amide de formule II est une pyrrolidone, de préférence substituée, et encore plus préférentiellement N-substituée.

6. Procédé selon la revendication 5, **caractérisé en ce que** ladite pyrrolidone est sélectionnée dans le groupe formé par la 2-pyrrolidone et la N-méthyl-2-pyrrolidone.

7. Suspension colloïdale de nanoparticules de fluorure d'élément alcalino-terreux fonctionnalisées susceptible d'être obtenue par le procédé selon l'une quelconque des revendications 3 à 6.

8. Suspension colloïdale selon la revendication 7, **caractérisée en ce que** lesdites particules ont un diamètre D50 mesurés par DLS (Dynamic Light Scattering) compris entre 5 nm et 30 nm, et de préférence entre 5 nm et 20 nm.

9. Suspension colloïdale selon la revendication 7 ou 8, **caractérisée en ce que** son extrait sec est d'au moins 20 %, de préférence d'au moins 25 %, et encore plus préférentiellement d'au moins 30 % massiques.

10. Utilisation d'une suspension colloïdale de particules de fluorure de calcium fonctionnalisées selon l'une quelconque des revendications 7 à 9 pour la fabrication d'un produit de traitement prophylactique ou curatif des dents.

11. Produit de traitement prophylactique ou curatif des dents **caractérisé en ce qu'**il comprend une suspension colloïdale de nanoparticules de fluorure de calcium fonctionnalisées selon l'une quelconque des revendications 7 à 9.

12. Produit selon la revendication 11, **caractérisé en ce qu'**il s'agit d'un vernis.

13. Produit selon la revendication 11, **caractérisé en ce qu'**il s'agit d'un vernis pour la prévention de la déminéralisation et/ou pour la reminéralisation stimulée par la salive, et/ou d'un vernis pour réduire la croissance de la plaque bactérienne.

14. Produit selon la revendication 11, **caractérisé en ce qu'**il s'agit d'un matériau de comblement dentaire.

## Patentansprüche

1. Verfahren zur Herstellung einer kolloidalen Suspension von funktionalisierten Nanopartikeln eines Fluorids eines Erdalkalielements, mit einem Trockenextrakt von mindestens 5 Ma.-% (vorzugsweise mindestens 10 % und bevorzugter mindestens 15 % und noch bevorzugter mindestens 25 %), wobei:
(a) eine Lösung eines Salzes des entsprechenden Erdalkalielements hergestellt wird,
(b) eine Lösung eines Charge-Transfer-Komplexes der Formel (I) hergestellt wird 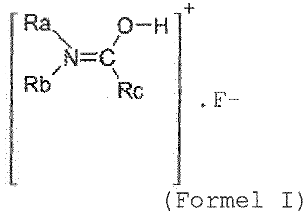 durch Reaktion eines Amids der Formel (II) 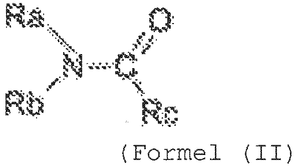 mit einer Fluoridionenquelle, vorzugsweise Flusssäure, Tetrabutylammoniumfluorid, Triethylamintrihydrofluorid, oder Fluorkieselsäure,
wobei: Ra und Rb, identisch oder unterschiedlich, jeweils unabhängig voneinander eine (C₁-C₆)Alkyl-, (C₃-C₇)Cycloalkyl-, Phenyl- oder Heterocycloalkyl-Gruppe mit 5 oder 6 Kettengliedern darstellt, wobei die Gruppe eventuell substituiert sein kann, oder aber Ra und Rb aneinander gebunden sind, und eine eventuell substituierte Alkylen-Kette bilden, die 2 bis 6 Kohlenstoffatome umfasst, und Rc ein Wasserstoffatom oder eine (C₁-C₆)Alkyl-, (C₃-C₇)Cycloalkyl-, Phenyl- oder Heterocycloalkyl-Gruppe mit 5 oder 6 Kettengliedern darstellt, wobei die Gruppe eventuell substituiert sein kann, oder aber Rb und Rc aneinander gebunden sind, und eine eventuell substituierte Alkylen-Kette bilden, die 2 bis 5 Kohlenstoffatome umfasst,
(c) die in Schritt (a) erhaltene Lösung des Salzes des entsprechenden Erdalkalielements und die in Schritt (b) erhaltene Lösung des Charge-Transfer-Komplexes der Formel (I) in einem Lösungsmittel oder einem Lösungsmittelgemisch in Kontakt gebracht werden,
(d) das in Schritt (c) erhaltene Gemisch eventuell unter Erwärmen reagieren gelassen wird, um eine kolloidale Suspension von Nanopartikeln eines Fluorids eines Erdalkalielements zu erhalten,
(e) die kolloidale Suspension von Nanopartikeln eines Fluorids eines Erdalkalielements optional vorzugsweise durch mindestens einen Zentrifugations-/erneuten Dispergierungszyklus in einem geeigneten Lösungsmittel gewaschen wird, bis der Überstand klar und farblos ist, wobei vorausgesetzt wird, dass das letzte Lösungsmittel zur erneuten Dispergierung in Abhängigkeit von dem Lösungsmittel zur Nutzung der kolloidalen Suspension von Nanopartikeln eines Fluorids eines Erdalkalielements ausgewählt wird,
wobei festzuhalten ist, dass die Schritte (a) und (b) umgekehrt werden können.

2. Verfahren nach Anspruch 1, wobei die Fluoridionenquelle im Schritt b eine Flusssäurelösung ist.

3. Verfahren zur Herstellung einer kolloidalen Suspension von funktionalisierten Nanopartikeln eines Fluorids eines Erdalkalielements, wobei:
(i) eine kolloidale Suspension von Nanopartikeln eines Fluorids eines Erdalkalielements unter Verwendung des Verfahrens nach Anspruch 1 oder 2 hergestellt wird,
(ii) an die Oberfläche der Nanopartikel eines Fluorids eines Erdalkalielements ein Molekül verpflanzt wird, das eine Struktur vom Typ A-Sp-Z aufweist, in der:
∘ A eine Funktion ist, die für das Anhaften des Moleküls an der Oberfläche sorgt,
° Sp eine Abstandhaltergruppe ist,
° Z eine chemische Funktion ist, die es ermöglicht, den Oberflächencharakter der Nanopartikel zu ändern,
(iii) die kolloidale Suspension von funktionalisierten Nanopartikeln eines Fluorids eines Erdalkalielements optional vorzugsweise durch mindestens einen Zentrifugations-/erneuten Dispergierungszyklus in einem geeigneten Lösungsmittel gewaschen wird, bis der Überstand frei von freien funktionalisierenden Molekülen ist, wobei vorausgesetzt wird, dass das letzte Lösungsmittel zur erneuten Dispergierung in Abhängigkeit von dem Lösungsmittel zur Nutzung der kolloidalen Suspension von funktionalisierten Nanopartikeln eines Fluorids eines Erdalkalielements ausgewählt wird.

4. Verfahren nach einem der Ansprüche 1 bis 3, **dadurch gekennzeichnet, dass** das Erdalkalielement aus Magnesium, Kalzium, Barium, Strontium ausgewählt wird.

5. Verfahren nach einem der Ansprüche 1 bis 4, **dadurch gekennzeichnet, dass** das Amid der Formel II ein Pyrrolidon, vorzugsweise substituiert, und noch bevorzugter N-substituiert ist.

6. Verfahren nach Anspruch 5, **dadurch gekennzeichnet, dass** das Pyrrolidon aus der Gruppe ausgewählt ist, die durch das 2-Pyrrolidon und das N-Methyl-2-Pyrrolidon gebildet ist.

7. Kolloidale Suspension von funktionalisierten Nanopartikeln eines Fluorids eines Erdalkalielements, die durch das Verfahren nach einem der Ansprüche 3 bis 6 erhalten werden kann.

8. Kolloidale Suspension nach Anspruch 7, **dadurch gekennzeichnet, dass** die Partikel einen Durchmesser D50, der durch DLS (Dynamic Light Scattering) gemessen wird, aufweisen, der zwischen 5 nm und 30 nm, und vorzugsweise zwischen 5 nm und 20 nm enthalten ist.

9. Kolloidale Suspension nach Anspruch 7 oder 8, **dadurch gekennzeichnet, dass** ihr Trockenextrakt mindestens 20 Ma.-%, vorzugsweise mindestens 25 Ma.-% und noch bevorzugter mindestens 30 Ma.-% ist.

10. Verwendung einer kolloidalen Suspension von funktionalisierten Kalziumfluoridpartikeln nach einem der Ansprüche 7 bis 9, zur Herstellung eines vorbeugenden oder kurativen Behandlungsprodukts für Zähne.

11. Produkt zur vorbeugenden oder kurativen Behandlung für Zähne, **dadurch gekennzeichnet, dass** es eine kolloidale Suspension von funktionalisierten Kalziumfluoridnanopartikeln nach einem der Ansprüche 7 bis 9 umfasst.

12. Produkt nach Anspruch 11, **dadurch gekennzeichnet, dass** es sich um einen Lack handelt.

13. Produkt nach Anspruch 11, **dadurch gekennzeichnet, dass** es sich um einen Lack zur Vorbeugung der Demineralisierung und/oder zur stimulierten erneuten Mineralisierung durch Speichel, und/oder um einen Lack zur Verringerung des Wachstums von bakteriellem Plaque handelt.

14. Produkt nach Anspruch 11, **dadurch gekennzeichnet, dass** es sich um ein Zahnfüllungsmaterial handelt.

## Claims

1. A method for preparing a colloidal suspension of functionalized alkaline earth element fluoride nanoparticles with a dry extract of at least 5% by mass (preferably at least 10%, and more preferably at least 15%, and even more preferably at least 25%), wherein:
(a) a solution of a salt of the corresponding alkaline earth element is prepared,
(b) a solution of a charge transfer complex of formula (I) is prepared 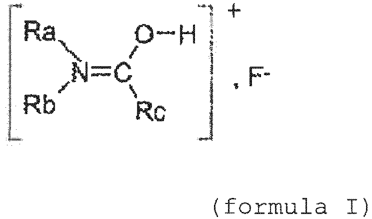 by reaction of an amide of formula (II) 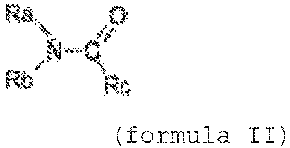 with a fluoride ion source, preferably hydrofluoric acid, tetrabutylammonium fluoride, triethylamine trihydrofluoride or fluorosilicic acid,
where: Ra and Rb, identical or different, represent, each independently of each other, a (C₁-C₆) alkyl, (C₃-C₇)cycloalkyl, phenyl or 5- or 6-membered heterocycloalkyl group, said group being optionally substituted, or else Ra and Rb are bonded to each other and form an alkylene chain, comprising 2 to 6 carbon atoms, optionally substituted, and Rc represents a hydrogen atom or a (C₁-C₆) alkyl, (C₃-C₇) cycloalkyl, phenyl or 5- or 6-membered heterocycloalkyl group, said group being optionally substituted, or else Rb and Rc are bonded to each other and form an alkylene chain, comprising 2 to 5 carbon atoms, optionally substituted,
(c) the solution of the salt of the corresponding alkaline earth element obtained in step (a) and the solution of the charge transfer complex of formula (I) obtained in step (b) are brought into contact, in a solvent or mixture of solvents,
(d) the mixture obtained in step (c) is allowed to react, optionally by heating, so as to obtain a colloidal suspension of alkaline earth element fluoride nanoparticles,
(e) optionally, the colloidal suspension of alkaline earth element fluoride nanoparticles is washed, preferably by at least one centrifugation/redispersion cycle in an appropriate solvent until the supernatant is clear and colourless, given than the last solvent of redispersion is selected depending on the solvent for using the colloidal suspension of alkaline earth element fluoride nanoparticles,
knowing that steps (a) and (b) can be reversed.

2. The method according to claim 1, wherein in step b, said fluoride ion source is a hydrofluoric acid solution.

3. The method for preparing a colloidal suspension of functionalised alkaline earth element fluoride nanoparticles wherein:
(i) a colloidal suspension of alkaline earth element fluoride nanoparticles is prepared by using the method according to claim 1 or 2,
(ii) a molecule having an A-Sp-Z type structure is grafted onto the surface of the alkaline earth element fluoride nanoparticles wherein:
∘ A is a function ensuring the attachment of the molecule to the surface,
∘ Sp is a spacer group,
∘ Z is a chemical function allowing changing the surface character of the nanoparticles,
(iii) optionally, the colloidal suspension of functionalised fluoride nanoparticles of the alkaline earth element is washed, preferably by at least one centrifugation/redispersion cycle in an appropriate solvent until the supernatant is free from free functionalising molecules, given than the last solvent of redispersion is selected depending on the solvent for using the colloidal suspension of functionalised alkaline earth element fluoride nanoparticles.

4. The method according to any one of claims 1 to 3, **characterised in that** the alkaline earth element is selected from magnesium, calcium, barium, strontium.

5. The method according to any one of claims 1 to 4, **characterised in that** said amide of formula II is a pyrrolidone, preferably substituted, and even more preferably N-substituted.

6. The method according to claim 5, **characterised in that** said pyrrolidone is selected from the group formed by 2-pyrrolidone and N-methyl-2-pyrrolidone.

7. A colloidal suspension of functionalised alkaline earth element fluoride nanoparticles obtainable by the method according to any one of claims 3 to 6.

8. The colloidal suspension according to claim 7, **characterised in that** said particles have a diameter D50 measured by DLS (Dynamic Light Scattering) comprised between 5 nm and 30 nm, and preferably between 5 nm and 20 nm.

9. The colloidal suspension according to claim 7 or 8, **characterised in that** the dry extract thereof is at least 20%, preferably at least 25%, and even more preferably at least 30% by mass.

10. A use of a colloidal suspension of functionalised calcium fluoride particles according to any one of claims 7 to 9, for the manufacture of a product for the prophylactic or curative treatment of teeth.

11. A product for the prophylactic or curative treatment of teeth, **characterised in that** it comprises a colloidal suspension of functionalised calcium fluoride nanoparticles according to any one of claims 7 to 9.

12. The product according to claim 11, **characterised in that** it is a varnish.

13. The product according to claim 11, **characterised in that** it is a varnish for the prevention of the demineralisation and/or for the remineralisation stimulated by saliva, and/or a varnish to reduce the growth of bacterial plaque.

14. The product according to claim 11, **characterised in that** it is a dental filling material.
